Europäisches Patentamt

European Patent Office

Office européen des brevets

(11) Publication number: **0 170 266 B1**

# EUROPEAN PATENT SPECIFICATION

(45) Date of publication of patent specification: **24.03.93**  (51) Int. Cl.5: **C12N 15/62**, C07K 15/00

(21) Application number: **85109592.7**

(22) Date of filing: **30.07.85**

(54) **Process for producing protein, and vector, recombinant DNA and transformant used therefor.**

(30) Priority: **30.07.84 JP 159703/84**
**26.12.84 JP 279585/84**

(43) Date of publication of application:
**05.02.86 Bulletin 86/06**

(45) Publication of the grant of the patent:
**24.03.93 Bulletin 93/12**

(84) Designated Contracting States:
**CH DE FR GB IT LI SE**

(56) References cited:
EP-A- 0 006 694      EP-A- 0 055 942
EP-A- 0 077 569      EP-A- 0 116 201
EP-A- 0 121 884      EP-A- 0 123 544
EP-A- 0 133 321      WO-A-84/04541
WO-A-86/00637

GENE, vol. 22, 1983, pages 229-235, Elsevier
Science Publishers, Amsterdam, NL; I.
PALVA et al.: "Secretion of interferon by
Bacillus subtilis"

(73) Proprietor: **WAKUNAGA SEIYAKU KABUSHIKI
KAISHA
2-14, Fushimi-Machi 4-Chome, Chuo-Ku
Osaka-shi Osaka-Fu(JP)**

(72) Inventor: **Miyake, Tetsuo
706-1, Kami Kotachi Kuoda-Cho
Takata-Gun Hiroshima-Ken(JP)**
Inventor: **Oka, Takanori
544-23, Yaguchi Koyo-Cho Asakita-Ku
Hiroshima-Shi Hiroshima-Ken(JP)**
Inventor: **Tamura, Gakuzo
2-17-12, Sanno Ota-Ku
Tokyo-To(JP)**
Inventor: **Yamasaki, Makari
4-102, Fuchu Daini Danchi 2-1-10, Harumi-
Cho
Fuchu-Shi Tokyo-To(JP)**
Inventor: **Yoda, Koji
5-22-15, Mejiro Toshima-Ku
Tokyo-To(JP)**

(74) Representative: **Kraus, Walter, Dr. et al
Patentanwälte Kraus, Weisert & Partner
Thomas-Wimmer-Ring 15
W-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 170 266 B1

PROCEEDINGS OF THE NATIONAL ACADEMY OF SCIENCES OF THE USA, vol. 77, no. 6, June 1980, pages 3369-3373, Washington, US; K. TALMADGE et al.: "Eukaryotic signal sequence transports insulin antigen in Escherichia coli"

JOURNAL OF BIOCHEMISTRY, vol. 97, no. 5, May 1985, pages 1429-1436, US; T. MIYAKE et al.: "Secretion of human interferon-alpha induced by using secretion vectors containing a promoter and signal sequence of alkaline phosphatase gene of Escherichia coli"

Oshuye et al. (1983), Nucl. Acids Res. 11(5), pp. 1283-94

Inouye et al. (1983), EMBO J. 2(1), pp. 87-91

Kadonaga et al. (1984), J. Biol. Chem. 259(4), pp. 2149-54

Grayeb et al. (1984), J. Biol. Chem. 259(1), pp. 463-67

**Description**

This invention relates to a process for producing a protein according to a genetic engineering method. More particularly, the present invention relates to a process for producing a protein according to a genetic engineering method which comprises utilizing a vector having a DNA gene which has been so designed that a gene coding for a desired exogenous or foreign protein is linked to and immediately after a gene coding for a signal peptide, and recovering efficiently the protein produced within a host cell and excreted out of the cell thanks to the action of the signal peptide.

Also, the present invention relates to a vector, a recombinant DNA and a cell which has been transformed by the recombinant DNA and which is thus capable of producing a desired protein to be used for the above process for production of a protein.

Techniques for producing desired genetic products in large amounts by utilizing the recombinant DNA techniques are being established as can be seen from the large number of reports and published patent specifications. With establishment of these techniques, genetic analyses are also carried out, and the knowledge that a series of peptides called signal peptides comprising 15 to 30 amino acid residues participate in secretion of the expressed proteins out of the host cells has also been found from among such analyses.

This knowledge concerning secretion of the expressed protein outside of cells is called the "Signal Hypothesis" (J. Cell Biol., 67, 835 (1975)), and experimental data supporting this hypothesis are being accumulated (Secretory Mechanisms, 9, Cambridge University Press, Cambridge (1979); Biochemistry, 52, 141 (1980), etc.). The outline and the function of signal peptides are described in, for example, Japanese Patent Laid-Open Publication No. 69897/1983, and signal peptides are recognized as participating in permeation of a protein through cell membrane.

At present, inventions utilizing signal peptides are disclosed in Patent Publications (Japanese Patent Laid-Open Publications Nos. 19092/1980, 45395/1980, 13786/1981, 145221/1981, 154999/1981, 192400/1982, 69897/1983, etc.). According to any of the methods disclosed in these Publications, the structural gene coding for the protein to be secreted from the host microorganism is cleaved at an appropriate restriction endonuclease recognition site, and an exogenous gene is linked thereto through a linker for matching a reading frame. As a result, the exogenous protein expressed from such a fused gene has a superfluous peptide at its N-terminal side. Therefore, it may be considered probable that such a superfluous peptide may interfere with secretion of the exogenous protein or have a deleterious effect on the biological activity of the exogenous protein.

On the other hand, according to Proc. Natl. Acad. Sci., USA, 77, 3988 - 3922 (1980) or Science, 219, 620 - 625 (1983), all of these utilize the signal peptides inherently possessed by the proteins to be expressed, and therefore utilization of such signal peptides may be limited only to excretion of the proteins having such native signal peptides.

Thus, the techniques of the prior art may be criticized as involving various problems, and it would be desirable to solve such problems.

Meanwhile, in the prior art, it was difficult to produce a protein with a relatively low molecular weight (e.g. Somatostatin [Science, 198, 1056 (1977)], but later it has been made possible to produce even a low molecular weight protein according to the hybridized protein method [Japanese Patent Laid-Open Publication No. 92696/1979], namely, the method in which a desired protein is produced as a hybridized protein with E. coli protein. However, for obtaining a desired protein according to the hybridized protein method, it is necessary to utilize the method in which the gene coding for methionine is inserted at a fusing point between the gene coding for the E. coli protein and the gene coding for the desired protein, and then the gene is expressed according to the required steps, followed by cleavage with cyanogen bromide which is specific for methionine to obtain the desired protein (Science supra) or the method in which the desired protein is obtained by the trypsin digestion specific for lysine or arginine [Nature, 285, 456 (1980)].

According to any one of these methods, depending on the amino acid composition of the desired protein, use of the method may be restricted. The reason for this is that in the case of the cyanogen bromide treatment, if methionine exists, or in the case of the trypsin digestion treatment, if lysine or arginine exists, in the amino acids constituting the desired protein, the protein will be cleaved at such an amino acid site, whereby a complete protein desired cannot be obtained.

On the other hand, for a protein having a molecular weight of a certain order or higher, it is possible to produce a desired protein containing no amino acid sequence derived from E. coli according to the direct expression method [Nature, 281, 544 (1979)]. According to the direct expression method, a gene having an initiation codon (ordinarily AUG coding for methionine) attached to the gene coding for a desired protein immediately therebefore is expressed subsequently to the required steps, but a product having methionine attached at the N-terminal may be produced depending on the kind of the protein {e.g., SV40t antigen [Nucleic Acids Res., 8, 4057 (1980)], β-globin [Proc. Natl. Acad. Sci. USA, 76, 5596 (1979)], etc.}, whereby no natural product can be obtained.

Thus, the process for producing a protein according to any of the above genetic engineering techniques may be restricted in its use depending on the desired protein to be obtained, and therefore it would be desirable to provide a process which can produce any desired protein without such restrictions as mentioned above.

Further, J. Bechwith et al suggest that about 2 x 10⁴ pores exist on the cell wall protein of E. coli, and these pores are common to excretable proteins (Cell., 24, 709 (1981)). Accordingly, it may be considered that, during permeation through the membrane, a plural number of excretable proteins will compete with each other, whereby extracellular excretion is lowered.

Thus, according to the process for production of a protein by use of the genetic engineering technique as described above, when secreting the desired protein out of the microorganism cell, competition may occur with excretable proteins other than the desired protein, and therefore it would be desirable to provide a method to permit the desired protein to be excreted efficiently out of the microorganism cell without being subject to such competition.

EP-A-0 077 569 relates to the isolation and utilization of a gene responsible for the formation and secretion of alkaline phosphatase of Escherichia coli. According to this document, a DNA gene which is derived from a gene encoding alkaline phosphatase (referred to as pho A) of Escherichia coli responsible for formation and secretion of proteins and having at least one of the promoter sequences, the Shine-Dalgarno sequence, the signal sequence and a combination thereof, is isolated and used.

Gene 22, pp. 229 to 235 (1983) refers to the secretion of interferon by Bacillus subtilis. Bacillus subtilis was transformed with a hybrid gene in which the sequence encoding the alpha-amylase signal peptide was joined by a linker to the sequence encoding mature human interferon-alpha2 (IFN-alpha2).

EP-A-0 006 694 relates to a method of making a selected protein. This is achieved by inserting DNA representing the selected protein or portion thereof into a bacterial gene, characterized by cleaving the bacterial gene for an extra-cellular or periplasmic carrier protein, inserting into the cleavage site by a recombinant step a non-bacterial DNA fragment which codes for the selected protein or portion thereof, transforming a bacterial host with the recombined gene, and culturing the transformed bacteria to excrete the selected protein or portion thereof.

EP-A-0 055 942 relates to a class of recombinant bacterial plasmid cloning vehicles for expression of exogenous genes in transformed bacterial hosts comprising a DNA insert fragment coding for the desired polypeptides linked in reading phase with one or more functional fragments derived from an outer membrane protein gene of any gram-negative bacterium. In a preferred embodiment as described in the specification, the functional fragments are derived from the lipoprotein gene of Escherichia coli which is linked to an exogenous gene.

Proc. Natl. Acad. Sci. USA relates to a series of plasmids with unique Pst restriction sites within or near the DNA that encodes the penicillinase signal sequence. Inserted DNA can be read in all three frames both within and immediately after the signal sequence. Pst-terminated DNA copies of the structural information for rat proinsulin and preproinsulin have been cloned into these plasmids, forming a large number of hybrid penicillinase (bacterial) and insulin (eukaryotic) signal sequences.

The EMBO J. 2, 87 - 91 (1983) teaches the fact that prolipoprotein signal peptidase of E. coli requires a cystein residue at the cleavage site for its function.

From J. Biol. Chem. 259, 463 - 467 (1984) it can be taken that nine amino acid residues at the NH₂-terminal of lipoprotein are sufficient for its modification, processing and localization in the outer membrane of Escherichia coli.

In Nucleic Acids Res. 11, 1283 - 1294 (1983), it is disclosed that the α-neo-endorphin was inserted into a DNA sequence downstream of an alkaline phosphatase gene (ca. 355 amino acids downstream of the N-terminus) and was expressed, but no secretion into the periplasm was found even though cleavage of the signal peptide was found. This is one of the instances where a signal peptide does not always result in the secretion of a fused protein.

An object of the present invention is to provide a solution to the point described above. We have found that this object can be accomplished by providing a process for producing a protein, which comprises utilizing a vector containing a DNA gene which has been so designed that a gene coding for a desired exogenous protein is linked to and immediately after a gene coding for a signal peptide, producing the desired protein in a host cell according to a genetic engineering technique, further permitting the desired protein to be excreted out of the cell thanks to the action of the signal peptide, and recovering the protein thus excreted.

Accordingly, the process for producing a protein according to the present invention comprises the steps of:

(a) culturing Escherichia coli cells which have been transformed with a recombinant DNA vector which comprises a gene encoding a natural signal peptide derived from Escherichia coli and a gene encoding the epidermal growth factor, the former gene being linked to the latter gene without any intervening nucleotide(s) therebetween;

(b) allowing a signal peptidase present in the Escherichia coli cells to cleave said gene product to permit secretion of the mature epidermal growth factor out of the cell; and

(c) recovering said epidermal growth factor in mature form.

In the preparation of the above recombinant DNA, it may sometimes be particularly preferable to use, for example, a recombinant plasmid, obtained as the result of linking a gene coding for a human epidermal growth factor or a 21-Leu epidermal growth factor to the gene coding for a signal peptide immediately after the end on the downstream side thereof, which has a base sequence comprising at least the gene coding for promoter, the gene coding for SD sequence, the gene coding for the signal peptide, and the gene coding for the above growth factors, and yet contains no gene coding for an excretable protein at the portion other than said base sequence portion.

Also, the present invention concerns a recombinant DNA vector and a cell transformed with the recombinant DNA capable of producing the above growth factors which can be used in the above process.

Thus, the recombinant DNA vector according to the present invention comprises a gene encoding a natural signal peptide derived from Escherichia coli and a gene encoding a human epidermal growth factor or a 21-Leu epidermal growth factor, the former gene being linked to the latter gene without any intervening nucleotide(s) therebetween, said gene product being cleaved by a signal peptidase present in the Escherichia coli cells thus permitting secretion of mature epidermal growth factor out of the cell.

Thus, the cell is capable of producing a human epidermal growth factor or a 21-Leu epidermal growth factor, wherein said cell is transformed with a recombinant DNA vector which comprises a gene encoding a natural signal peptide derived from Escherichia coli and a gene encoding the epidermal growth factor, the former gene being linked to the latter gene without any intervening nucleotide(s) therebetween said gene product being cleaved by a signal peptidase present in the Escherichia coli cells thus permitting secretion of mature epidermal growth factor out of the cell.

The present invention thus relates to a process for producing a protein according to a genetic engineering technique characterized, in order to permit the protein derived from an exogenous gene to be excreted out of the cell by a signal peptide, by the utilization of a vector comprising a gene encoding a natural signal peptide derived from Escherichia coli and a gene encoding an epidermal growth factor, the former gene being linked to the latter gene without any intervening nucleotide(s) therebetween, said gene product being cleaved by a signal peptidase present in the Escherichia coli cells thus permitting secretion of mature epidermal growth factor out of the cell.

According to a preferred embodiment of the present invention, for facilitating linking of the structural gene of the desired growth factor protein to the signal peptide gene immediately after the end on the downstream side thereof, the gene coding for the signal peptide employed has a restriction endonuclease recognition site artificially created, at least one of base pairs of a gene comprising the signal peptide gene as being at least a part of the constituent members.

In creating of such a site, it is possible to utilize the fact that there is degeneracy in the codon comprising DNA base pairs. Thus, if the restriction endonuclease recognition/cleavage site is cleaved with the restriction endonuclease, when the cleavage side exists in contact with the signal peptide gene DNA at its end on downstream side, by preparing an exogenous gene having formed an end complementary to the end cleaved by the restriction endonuclease on the upstream side thereof and linking this gene to the signal peptide gene at the above cleaved end, the exogenous gene can be linked directly to the signal peptide gene on its downstream side.

On the other hand, when the cleavage site of the signal peptide gene exists upstream of the downstream side end, the portion of the gene on downstream side of the cleavage site can be synthesized and linked to the upstream side of the exogenous gene to prepare a fragment, after which the fragment is linked similarly as described above, whereby the signal peptide gene once cleaved can be restored on both chains of DNA, and also a structure having the exogenous gene linked directly to its down stream side can be realized. Further, when the cleavage site is such that a cohesive end is produced upon enzymatic digestion, the single strand DNA part of the cohesive end thus produced will be hydrolyzed to form a blunt end by means of S1 nuclease or DNA polymerase, which blunt end will in turn be linked to a gene having an exogenous gene prepared as described above.

Consequently, since the desired protein is linked immediately after the signal peptide, through the function of the signal peptide as mentioned above, the protein after expression within the host cell can be excreted out of the cell. In the excretion of the desired protein, the signal peptide is hydrolyzed by a membrane enzyme, viz. signal peptidase, and therefore no superfluous amino acid is linked to the protein to produce a complete mature protein.

Thus, the process for producing a human epidermal growth factor or a 21-Leu epidermal growth factor of the present invention makes possible the production of the above growth factors as a mature protein by utilizing skillfully the function of secretion out of the host cell by a signal peptide. The expression "secretion out of the host cell" as herein used means migration of the above growth factors from within the host cell at least to the periplasm (space between the cytoplasmic membrane and the cell wall) when the host is a Gram-negative microorganism such as E. coli.

The process for producing a human epidermal growth factor or a 21-Leu epidermal growth factor according to the present invention comprises incorporating a desired gene into a vector having secretion function, then transforming a host with the use of the gene on the vector, culturing the host microorganism, and thereafter recovering the desired protein.

Furthermore, the present invention concerns the recombinant DNA vector and the cell capable of producing the desired growth factor in which the recombinant DNA is incorporated.

Accordingly, the present invention, while accomplishing the above objects, has the following advantages:

(a) Purification of the human epidermal growth factor or the 21-Leu epidermal growth factor is simple. In the prior art, after growth of the host microorganism, the host microorganism was lysed, and the desired protein was obtained by extraction from miscellaneous substances inherently possessed by the microorganism, whereby an enormous amount of labor was required and some substances could not be easily purified. According to the process for the production of the above growth factors of the present invention, through the function of the signal peptide as described above, the growth factor produced is excreted out of the cells, and the desired substance can be easily identified in and recovered from the culture medium because the culture medium for growth of the microorganism is constituted of known components.

(b) The growth factor produced can be protected from decomposition with peptidase. That is, various peptidases (proteases) exist within the microorganism cell and proteins produced in and unnecessary to the cell will rapidly be hydrolyzed. If the above growth factors are immediately permitted to be excreted out of the microorganism cell without remaining in the microorganism cell, the above growth factors can be protected from digestion by the above hydrolyzing enzymes.

According to the hybridized protein method, for obtaining a desired protein in pure form, a superfluous protein must be cleaved by the cyanogen bromide treatment specific for methionine (Science, 198, 1056 (1977)), or the trypsin digestion specific for lysine or arginine must be conducted (Nature, 285, 456 (1980)). In such cases, when these amino acids are contained in the amino acid composition of the desired protein, cleavage will also occur at that portion, whereby the desired protein cannot be obtained in its complete form. On the other hand, when a protein is to be produced according to the direct expression method (Nature, supra), the gene is required to have an initiation codon (methionine) at its N-terminal, and the protein obtained may sometimes have methionine at its N-terminal (see Japanese Patent Laid-Open Publication No. 68399/1981). Since it is technically difficult to remove such terminal methionine by decomposition with the cyanogen bromide treatment, the protein produced will consequently become different in nature from the natural product. In contrast, when the exogenous gene is expressed in the host microorganism with the use of the recombinant plasmid according to the present invention as the vector, the protein once produced as the fused or hybridized protein with the signal peptide is cleaved specifically at the signal peptide portion with the signal peptide within the host microorganism and excreted as a mature protein with a desired composition out of the host microorganism cell.

Further, a preferred embodiment of the process for producing a human epidermal growth factor or a 21-Leu epidermal growth factor of the present invention is a process for producing a protein by a genetic engineering technique, utilizing a recombinant plasmid, obtained as the result of linking a gene coding for the desired growth factor to the gene coding for a signal peptide immediately after the end on the downstream side thereof, which contains a base sequence comprising at least the gene coding for promoter, the gene coding for SD sequence, the gene coding for the signal peptide, and the gene coding for the desired growth factor and yet contains no gene coding for an excretable protein at the portion other than the base sequence portion.

According to this process, the desired growth factor can be excreted efficiently out of the microorganism cell. That is, by use of a plasmid in which no gene coding for excretable proteins other than the gene coding for the desired growth factor exists, the desired protein produced can efficiently be excreted out of the microorganism cell without competition with the already existing excretable proteins derived from the plasmid.

Fig. 1 is a schematic diagram showing the base sequence near the end of the signal peptide (1) of pTA529 and indicates the restriction endonuclease recognition site (2), the restriction endonuclease cleavage site (broken line) and the signal peptide cleavage point (4);

Fig. 2 is a flow chart of the production of pTA1529;

Fig. 3 is a schematic diagram showing the base sequence of the structural gene of the hUG (and Leu-21-hUG) synthesized;

Fig. 4 is a flow chart of the production of the structural gene of pTA1522 (pTA1523 when containing 21-Leu-hUG) containing the structural gene of hUG (21-Leu-hUG);

Fig. 5 is a graph indicating the results of the RRA method;

Fig. 6 is a Sephadex® G-50 column chromatogram;

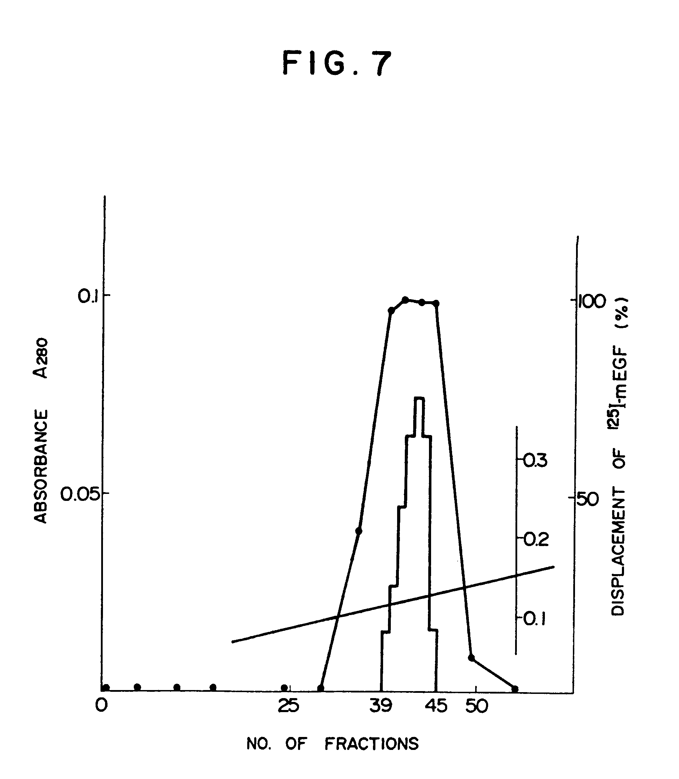

Fig. 7 is a DEAE Sepharose DE-52 column chromatogram;

Fig. 8 is a graph indicating an elution pattern by HPLC;

Fig. 9 is a flow chart of the production of pTA1502;

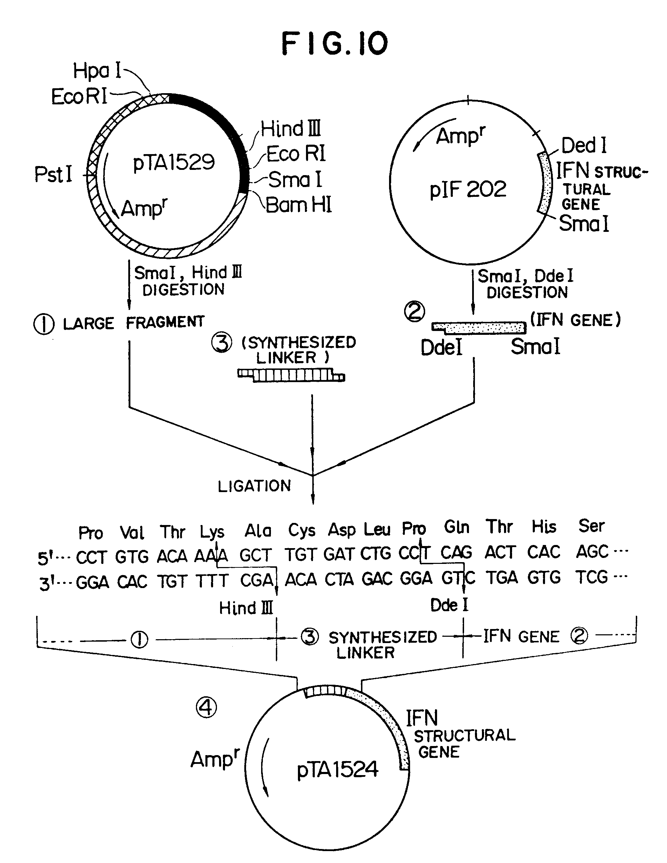

Fig. 10 is a flow chart of the production of pTA1524;

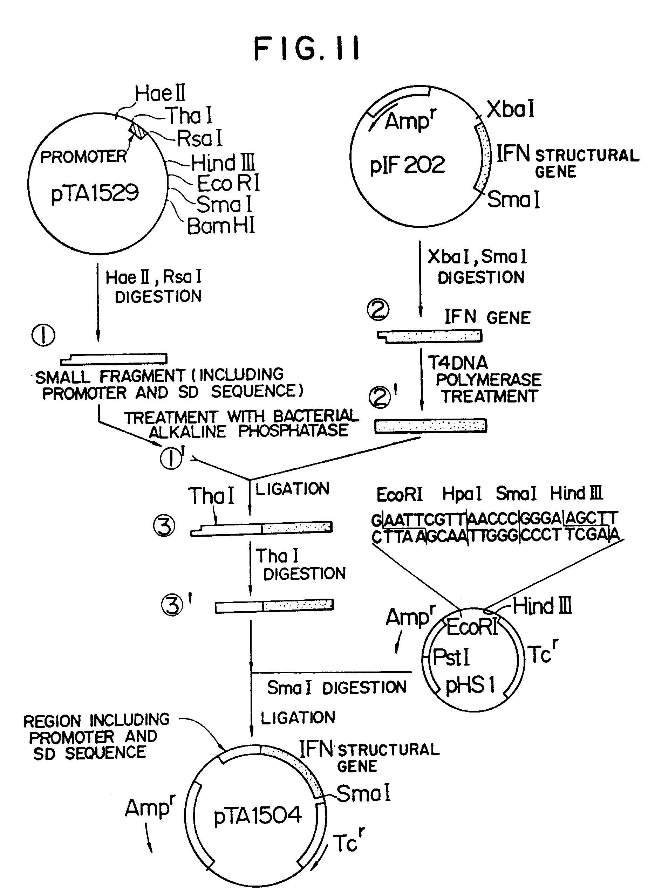

Fig. 11 is a flow chart of the production of pTA1504;

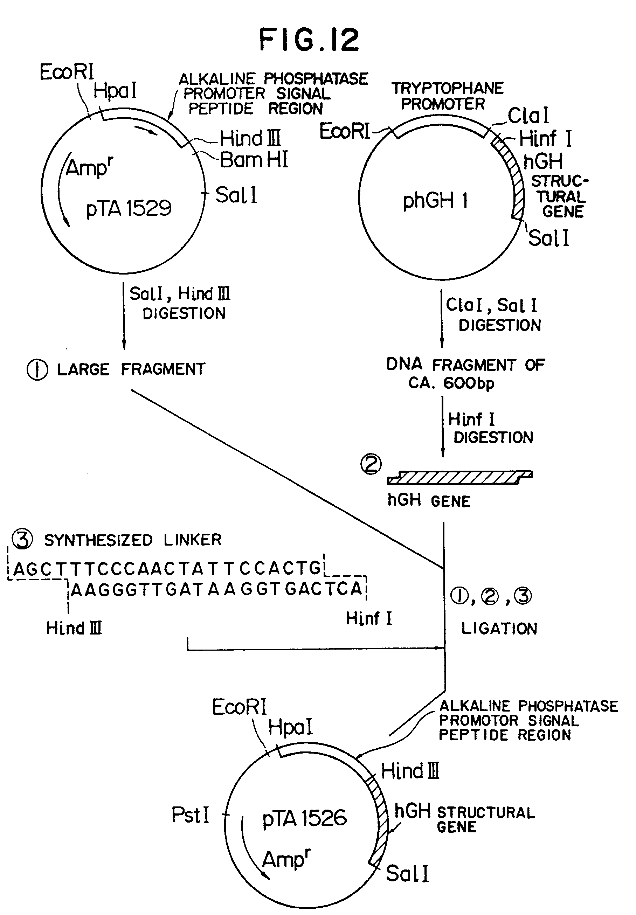

Fig. 12 is a flow chart of the production of pTA1526;

Figs. 13 - 15 are flow charts of the production of pTA1632;

Fig. 16 is a flow chart of the production of pTA2529;

Fig. 17 is a flow chart of the production of pTA2539;

Fig. 18 is a flow chart of the production of pTA2522 containing the structural gene of hUG; and

Fig. 19 is a flow chart of the production of pTA2532 containing the structural gene of hUG.

## Gene coding for desired protein

The gene (structural gene) coding for the desired protein contemplated includes those of human epidermal growth factor and 21-Leu epidermal growth factor which may be either prepared from a natural product (chromosome DNA) or synthesized.

The methods for preparation of the genes coding for these desired proteins are described in various textbooks, literatures, laid-open or published patent specifications, of which the principal ones are enumerated below.

(1) Methods for preparing a desired gene directly from chromosome DNA [Proc. Japan Acad., 55B, 464 (1979), Japanese Patent Laid-Open Publications Nos. 13099/1982, 166992/1982, 208994/1982 and 13599/1983);

(2) Methods in which messenger RNA (hereinafter called mRNA) is prepared, and a desired gene is prepared after the known necessary steps on the basis of the mRNA (Japanese Patent Laid-Open Publications Nos.56684/1983, 2998/1981, 36499/1981, 63996/1981, 85296/1981, 104897/1981, 131522/1981, 131598/1981, 150100/1981, 154499/1981, 158793/1981, 158799/1981, 24400/1982, 141287/1982, 171999/1982, 174085/1982, 206700/1982 and 56684/1983);

(3) Methods for preparing a gene coding for a desired protein by chemically synthesizing a DNA on the basis of an amino acid sequence of a protein produced by the desired gene (Japanese Patent Laid-Open Publications Nos. 122096/1982, 200343/1982, 200400/1982 and 10600/1983).

From among the above methods, the most suitable for reference is determined according to the gene coding for the desired protein. In the modes of practice of the present invention set forth below, the chemically synthesized structural gene of human urogastrone (hereinafter called hUG) was employed. The details of its manipulation are disclosed in Japanese Patent Laid-Open Publication No. 66992/1985.

## Vector having secretion function

In genetic manipulation, vector refers to a vehicle DNA having a role of transferring an exogenous gene (a gene coding for a desired protein) into a host cell and proliferating the exogenous gene in the cell, and a plasmid or a phage existing outside of chromosome has the above proliferating function in a microorganism cell.

In the present invention, as the vector having the above function and the secretion function, it is possible to use the above plasmid or phage in which a DNA gene having the secretion function as previously proposed by us (Japanese Patent Laid-Open Publication No. 30687/1985) is incorporated.

A typical example of such a vector is the vector plasmid pTA1529 employed in the present invention. The plasmid pTA1529 was prepared from pTA529 (made from pYK 283 [deposited as E. coli K12C600-(pYK283) (FERM-BP No.556)] according to the method disclosed in Japanese Patent Laid-Open Publication No. 30687/1985) and pHSI. (This plasmid was made by digesting pBR322 with restriction enconucleases EcoRI and HindIII and substituting the EcoRI-HindIII portion with the following synthetic linker:

```
 AATTCGTT AACCC GGGA
    GCAA TTGGG CCCTTCGA
  EcoRI HpaI  SmaI  HindIII
```

See Japanese Patent Laid-Open Publication No.71692/1984 about its details.) (As to the construction procedure, see the experimental examples presented hereinafter.)

Thus, the gene coding for the signal peptide in the above mode of practice is derived from an alkaline phosphatase. Otherwise, there may be employed any desired gene derived from $\beta$-lactamase (lipoprotein, etc.). Such a gene may be procured either from a natural product or synthesis, as a matter of course. When it is to be procured by way of synthesis, the gene can be designed and synthesized by appropriate selection of the codons corresponding to the amino acids, if necessary.

Recombinant DNA

1) Construction:

A recombinant DNA is constructed by incorporating the gene coding for the desired protein at an appropriate position in a vector which is devised to express and excrete the exogenous (desired protein) gene. The incorporation manipulation itself can be performed according to a conventional manner known in the field of molecular biology. Typical specific methods are described in the experimental examples given hereinafter.

2) Linker:

In incorporating the structural gene into a vector, use of various synthesized linkers is an effective measure in recombinant DNA technique for the purpose of matching in reading frame or introducing desired cleaved fragments of restriction endonuclease or ribosome binding site.

In one mode of practice of the present invention, a linker is also used for the purpose of matching in reading frame. That is, a synthetic linker is used for the purpose of utilizing the secretion function of the signal peptide and obtaining the desired protein without attachment of superfluous amino acids. (As for practical application examples, see the examples given hereinafter.)

Synthesis of a linker can be accomplished by any desired method in which each of + - strands divided into several fragments are chemically synthesized, and the respective segments are ligated to each other. Examples of the synthetic method for fragments are the solid phase method (Nucleic Acids Research, 8, 5491 (1980)), the liquid phase method and respective diester method (Science, 203, 614 (1976)), the triester method (Science, 198, 1056 (1977)), and the method employing an enzyme (J. Biol. Chem., 241, 2014 (1966)). On the points of synthetic time, conversion, purification, etc., the use of the solid phase method by way of the triester method is preferred.

3) Judgement of direction:

The direction of the gene coding for the desired protein incorporated in the vector can be judged by cleaving the specific site included within the structural gene with an enzyme capable of recognizing the site, cleaving a specific site outside of the structural gene with another enzyme and analyzing the size of the fragment obtained.

Recombinant plasmid

In manipulation of genes, plasmid refers to a genetically stable genetic factor which proliferates autonomously and independently of the host chromosome in the cytoplasma of a microorganism and is

EP 0 170 266 B1

useful as a vehicle DNA having a role of introducing an exogenous gene (a gene coding for the desired protein) into the host cell and proliferating the exogenous gene in the cell.

The plasmid according to one mode of practice of the present invention has the above function and also a secretion function, in which no gene coding for excretable proteins other than the gene coding for the exogenous protein to be incorporated exists at all.

A plasmid having a secretion function as herein mentioned has a base sequence comprising at least a gene coding for a promoter, a gene coding for a SD sequence, a gene coding for a signal peptide, and a gene coding for the desired exogenous protein.

(a) Promoter:

In a plasmid, the promoter is one of the most important regions related to expression efficiency of the plasmid.

The gene coding for the promoter to be used in the present invention, namely the DNA region to be recognized by RNA polymerase for transcription, may be procured from a natural chromosome DNA, or it is also possible to use a synthesized one, since the base sequences of a large number of promoter genes have already been determined. Further, an entirely new promoter may be synthesized and used. (See Japanese Patent Laid-Open Publication No.71692/1984.)

Among these, preferable promoter regions are those with strong promoting action, such as Trp-promoter,rec A-promoter, PL-promoter, and lac-promoter. [Protein-Nucleic Acid-Enzyme, 26, 443 (1981)]. In the examples set forth hereinafter, a promoter derived from an alkaline phosphatase is employed.

(b) SD sequence:

This is also called the Shine-Dalgarno sequence which refers to the sequence of mRNA existing immediately before the translation initiation codon (AUG or GUG) and linked to the 3'-end sequence of 16sRNA of ribosome, which can either be procured from nature or chemically synthesized, similarly as the promoter.

(c) Signal peptide:

The signal peptide in the plasmid in the present invention is preferably one possessing one restriction endonuclease recognition site for, e.g. Hind III, Nael, etc. which has been created artificially in the gene containing the DNA portion corresponding to the gene coding for the signal peptide, as we have described in Japanese Patent Laid-Open Publication No. 30687/1985, and which has at least one of the base pairs of the DNA as at least a part of the constituent members. (See the specification of the above application for details.) The gene coding for such a signal peptide is as described above.

The recombinant plasmid to be described in one mode of practice of the present invention is obtained by cleaving away a gene coding for an excretable protein other than the desired exogenous protein existing in the plasmid having the secretion function as described above, for example, an ampicillin resistant gene (hereinafter indicated as Amp$^r$), in a conventional manner. According to necessity, after removal of the gene coding for the excretable protein, non-excretable drug resistant marker (drug resistant marker coding for no excretable protein), for example, a kanamycin resistant gene (hereinafter indicated as Km$^r$), and a tetracycline resistant gene (hereinafter indicated as Tc$^r$) can be introduced as the selection marker.

A typical example of such a plasmid is the plasmid pTA2529 used in the present invention. The plasmid pTA2529 was constructed by cleaving the pTA1529 as described above with restriction endonucleases PstI and AatII in a conventional manner to remove Amp$^r$, which step was followed by introduction of a non-excretable drug resistant marker, for example, kanamycin resistant gene (Km$^r$) thereinto. (As to the construction operation, see the experimental examples set forth hereinafter.)

The recombinant plasmid, which is one mode of practice of the present invention, can be constructed by incorporating the gene coding for a desired protein at an appropriate site in the plasmid as prepared above which has been devised to express the exogenous (desired protein) gene and excrete the protein. The incorporation operation itself can be performed by following a known procedure known in the field of molecular biology. As to the details of the method, reference is made to the examples presented hereinafter.

The excretable protein other than the desired protein as mentioned in one mode of practice of the present invention refers to a protein excretable out of the microorganism cell encoded by the plasmid used as the vector other than the gene coding for the desired exogenous protein to be incorporated, as

9

exemplified by $\beta$-lactamase (Ann. Rev. Microbiol., 36, 435 (1982)). The gene coding for such a protein is called, in the present invention, the gene coding for an excretable protein.

Cell producing desired exogenous protein/transformant

In the present invention, the cell producing the desired exogenous protein is a microorganism cell transformed with the above recombinant DNA (transformant). The microorganism into which the above recombinant can conveniently be transferred (hereinafter sometimes referred to as host microorganism) is Escherichia coli, in which the recombinant DNA as constructed above is proliferable, and of which genetic characteristics are clarified in detail.

1. Host microorganism: Specific examples of such host microorganisms are E. coli strains belonging to the genus Escherichia, K12C600 (FERM-BP No.115), K12YK537 (FERM-BP No. 822) and XA35 (FERM-BP No.116).

On the other hand, in one mode of practice of the process for production of a protein of the present invention, a recombinant plasmid is employed, in which the gene coding for the desired exogenous protein is linked to the gene coding for the signal peptide immediately thereafter, and the existing gene coding for excretable protein other than the gene coding for the exogenous protein is artificially removed. This is done for further enhancing the extracellular secretion of the desired protein, and the host microorganism to be used in this case is one in which expression of excretable protein derived from the host chromosome is suppressed or defective. A specific example of such a host microorganism is an E. coli strain YK537 belonging to the genus Escherichia (supra).

The excretable protein derived from the chromosome as mentioned herein refers to a protein excretable out of the cell encoded by the chromosome gene of the host microorganism, as exemplified by alkaline phosphatase, cell wall principal lipoproteins, cell wall OmpC protein, cell wall ompF protein, and cell wall OmpA protein, [Ann. Rev. Microbiol., 36, 435 (1982)].

2. Transformant:

The cell producing the desired exogenous protein or transformant as mentioned in the present invention refers to a microorganism (host microorganism) transformed by transfer of the above secretion vector (recombinant DNA) in which the gene coding for a desired exogenous protein is incorporated, and the host microorganism is one in which the vector is proliferable.

The transformation operation itself may be conducted according to any of the conventional methods known in the field of molecular biology. For instance, the Cushner method will be used when transformation of E. coli is desired [Genetic Engineering 1978, 17 (1978)].

The strain transformed by the transforming operation can be selected by means of an index of a marker of the recombinant DNA (drug resistance, nutrient demand, etc.) created by transfer of the recombinant plasmid into the transformant.

A typical example of such a transformant is the transformant K12C600 (pTA1524) obtained by transformation of the E. coli K12C600 with pTA1524 (as described in detail hereinafter). This transformant is a microorganism strain different from the host microorganism K12C600 in the following properties:

Amp$^r$, Tc$^s$.

Therefore, with these properties as the index, the transformant can be selected.

Another typical example of the transformant in the present invention is the transformant obtained by transformation of the E. coli K12YK537 with pTA2532 (Ref.: experimental example presented hereinafter). This transformant is a microorganism strain different in the property of kanamycin resistance (Km$^r$) from the host microorganism K12YK537. Therefore, the transformant can also be selected with this property as the index.

Production and recovery of desired protein

The desired protein can be produced by culturing the host microorganism (namely the above transformant) in a conventional manner. Specific details of the culturing method are disclosed in the experimental examples given hereinafter.

For recovery of the desired protein from microorganism cells or the culture broth when the host microorganism is a Gram-negative microorganism such as E. coli, since the desired protein is secreted into periplasm through the secretion function of the signal peptide, the desired protein can readily be recovered after it is released from the periplasm outside of the cell wall (into the culture medium) according to the osmotic shock method [J. Biol. Chem., 240, 3685 (1965)].

Details of the method for culturing and recovery of the desired protein after the osmotic shock method in the case of the host microorganism of E. coli, are described in the experimental examples set forth hereinafter.

EXPERIMENTAL EXAMPLES

Reference Examples

Preparation of a vector having secretion function was carried out as follows.

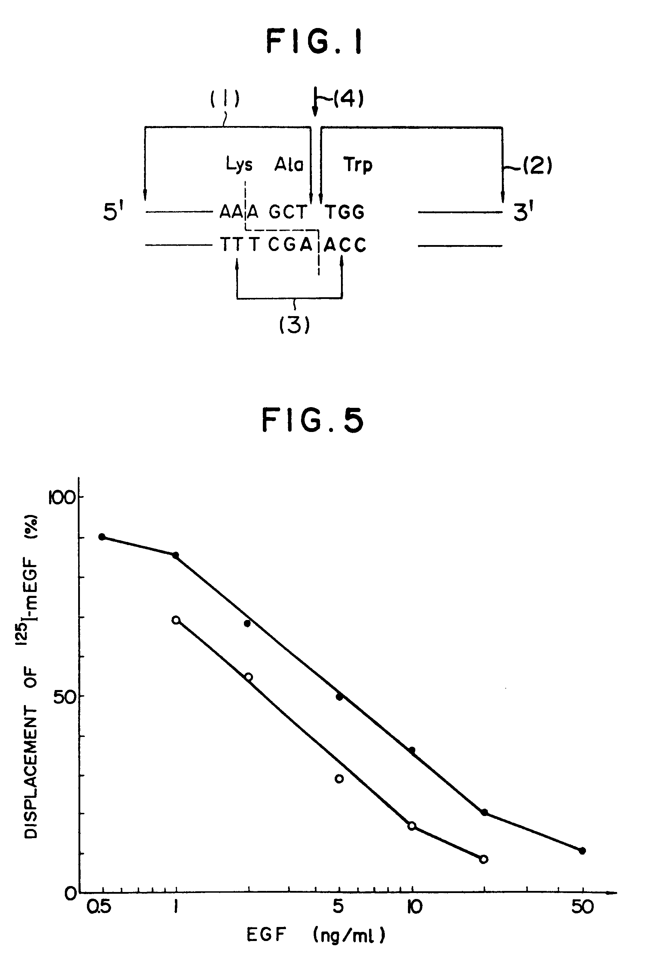

As the vector to be used in the process of the present invention, the plasmid pTA529 previously proposed by us [Japanese Patent Application No.140748/1983; having the terminal codon on the downstream side of the DNA portion corresponding to the gene coding for the signal peptide of a plasmid pYK283, deposited as E. coli K12C600 (pYK283) at the Fermentation Research Institute, Agency of Industrial Science & Technology (FERM-BP No. 556), and the initial codon of the DNA portion directly linked thereto on its downstream side as shown in Fig. 1] was further improved to prepare a vector pTA1529 having a still stronger self-replicating ability.

In this connection, Fig. 1 shows a part of the DNA gene comprising a double-stranded DNA, in which A, G, C and T represent adenine, guanine, cytosine and thymine, respectively, and Lys, Ala and Trp represent lysine, alanine and tryptophane, respectively. The region (1) in the double-stranded DNA is the signal peptide gene portion, the region (3) the recognition site of the restriction endonuclease Hind III, and the broken line shows the HindIII cleavage site. The region (2) is the DNA portion linked to the signal peptide gene DNA immediately thereafter on the downstream side.

Preparation of pTA1529 was carried out following the procedure as described below.

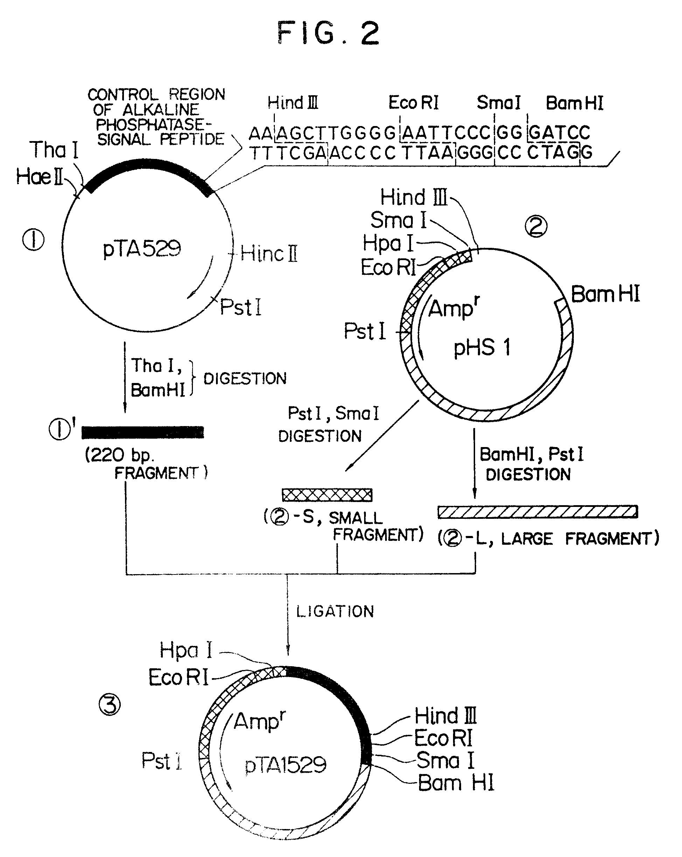

In 50 $\mu$l of a reaction mixture [50 mM Tris-hydrochloride buffer (hereinafter referred to Tris-HCl) (pH 8.0), 10 mM magnesium chloride (hereinafter written MgCl$_2$)], 5 $\mu$g of pTA529 (Fig. 2 ①) was hydrolyzed with 10 units of a restriction endonuclease Tha I [BRL Co.] (hereinafter referred to as Tha I) at 60°C for one hour. Then, ethanol precipitation was carried out, and the precipitated fraction obtained was hydrolyzed in 50 $\mu$l of a reaction mixture [10 mM Tris-HCl (pH 8.0), 7 mM MgCl$_2$, 100 mM sodium chloride (hereinafter referred to as NaCl)] with 4 units of a restriction endonuclease BamHI [Takara] (hereinafter written BamHI) and was then subjected to polyacrylamide gel electrophoresis to obtain a DNA fraction of about 220 base pairs (hereinafter abbreviated as bp) (represented by

in Fig. 2) ①' containing the desired alkaline phosphatase-signal peptide region.

On the other hand, 10 $\mu$g of the plasmid pHSI (see Japanese Patent Laid-Open Publication No. 71692/1984) was hydrolyzed with 4 units of a restriction endonuclease PstI [Takara] (hereinafter referred to as PstI) in 50 $\mu$l of a reaction mixture [10 mM Tris-HCl (pH 7.5), 10 mM MgCl$_2$ and 100 mM NaCl] at 37°C for one hour. Then, ethanol precipitation was carried out, and the precipitate obtained was divided into two aliquots, one of which was hydrolyzed with 40 units of BamHI in 50 $\mu$l of the above reaction mixture, which step was followed by agarose gel electrophoresis to obtain a DNA fragment of 330 bp (represented by

in Fig. 2) ②-S. On the other hand, the other aliquot of the ethanol precipitate was hydrolyzed similarly with 4 units of a restriction endonuclease SmaI [Takara] (hereinafter referred to as SmaI) in 50 $\mu$l of a reaction mixture [6 mM Tris-HCl (pH 8.0), 20 mM potassium chloride (hereinafter referred to as KCl), 6 mM MgCl$_2$] at 37°C for one hour, after which agarose gel electrophoresis was carried out to obtain a DNA fragment of 700 ba (represented by

in Fig. 2) ②-L.

The three DNA fragments obtained in the above operations (①', ②-S and ②-L) were allowed to react with the use of 30 units of T4 DNA ligase [Takara] in 30 $\mu$l of a reaction mixture [20 mM Tris-HCl (pH 7.5), 10 mM MgCl$_2$, 10 mM dithiothreitol (hereinafter referred to as DTT), 0.5 mM adenosine triphosphate (hereinafter referred to as ATP)] at 14°C for 16 hours. After the reaction, the E. coli K12C600 was transformed with the reaction product [already deposited as E. coli K12C600 (FERM-BP No. 115)] to obtain a transformed strain containing the desired plasmid (pTA1529). From the strains thus obtained, plasmids were prepared (the details of the preparation method are disclosed in Japanese Patent Laid-Open Publication No. 30687/1985), and the base sequences at the linking portions of the respective DNA fragments were confirmed according to the Maxam-Gilbert method [Proc. Natl. Acad. Sci. USA, 74, 560 (1977)].

Example 1

Human urogastrone (hUG/EGF) and 21-leuchine-human urogastrone (21-Leu-hUG) in which methionine of the 21st amino acid in the human urogastrone was replaced with leucine were produced according to the process of the present invention and recovered. Since the procedures for preparation of hUG and 21-Leu hUG are similar, the preparation of hUG will be described primarily.

Synthesis of the structural gene of hUG (and 21-Leu-EGF)

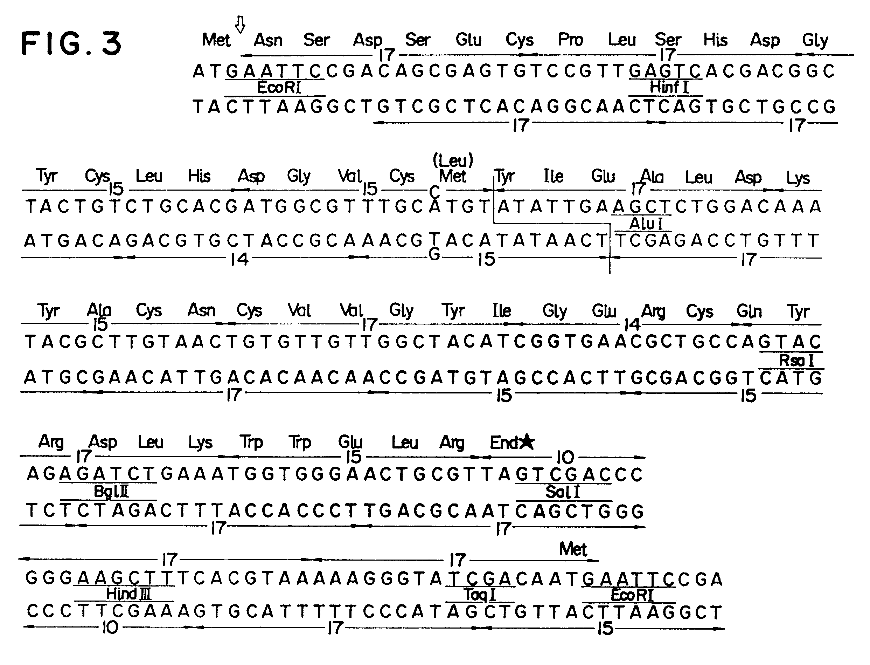

Synthesis of the structural gene of hUG (and 21-Leu-EGF) was carried out according to the process as disclosed in Japanese Patent Laid-Open Publication No. 28994/1985. More specifically, fragments of chain lengths of 10 - 17 were previously synthesized, and then these synthetic fragments were subjected to linking reactions for respective blocks, followed finally by binding of these blocks to synthesize a complete hUG structural gene. The base sequence of the hUG structural gene is as shown in Fig. 3, in which the mark ↓ indicates the initiation point, and the mark * the termination point. In Fig. 3, Met, etc. denote amino acids such as methionine, etc., and A, T, C and G denote bases, indicating abbreviations for adenine, thymine, cytosine and guanine, respectively, as approved in this field of the art. Further, the numerals in Fig. 3 indicate the number of the bases in the fragment, and the notation such as EcoRI indicates the restriction endonuclease recognition site. In the base sequence of 21-Leu-hUG, the 21st codon is changed to that coding for Leu in place of Met, as written (Leu) above Met in Fig. 3, the base sequence being otherwise the same as in hUG.

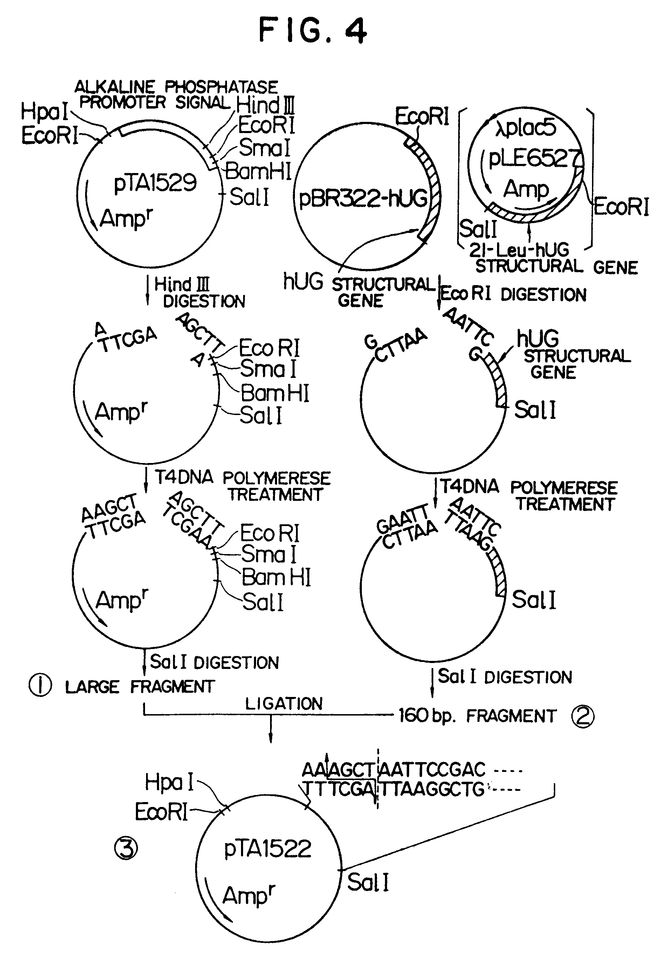

Preparation of a plasmid containing the hUG (21-Leu-hUG) gene (see Fig. 4):

Hydrolysis of 5 $\mu$g of pTA1529 was conducted in 50 $\mu$l of a buffer [10 mM Tris-HCl (pH 7.5), 10 mM MgCl$_2$, 50 mM NaCl] with 4 units of a restriction endonuclease HindIII [Takara] (hereinafter referred to as HindIII) at 37°C for one hour. The precipitate obtained by ethanol precipitation was treated in 30$\mu$l of a reaction mixture [67 mM Tris-HCl (pH 8.8), 16.6 mM ammonium sulfate (hereinafter referred to as (NH$_4$)$_2$SO$_4$), 6.7 mM MgCl$_2$, 10 mM 2-mercaptoethanol, 6.7 $\mu$M ethylenediaminetetraacetic acid (hereinafter referred to as EDTA), 0.66 mM each of dATP, dCTP, dGTP and TTP) with one unit of T4 DNA polymerase at 37°C for 15 minutes. Subsequently, ethanol precipitation was carried out, and the resultant precipitate was hydrolyzed in 50 $\mu$l of a reaction mixture [6mM Tris-HCl (pH 8.0), 6 mM MgCl$_2$ and 150 mM NaCl] with 4 units of a restriction endonuclease SalI [Takara] (hereinafter referred to as SalI) at 37°C for one hour. After completion of the reaction, a DNA fragment of 3,900 bp (Fig. 4 ①) was obtained by agarose gel electrophoresis.

Hydrolysis of 5 $\mu$g of the plasmid pBR322-hUG [obtained by incorporating the fragment of the hUG structural gene synthesized as described above formed by digestion with EcoRI and SalI into the pBR322 (deposited as E. coli K12C600(pBR322) (FERM-P No.235) digested with EcoRI and SalI) was conducted in 50 $\mu$l of a reaction mixture [100 mM Tris-HCl (pH 7.5), 50 mM NaCl and 50 mM MgCl$_2$] with 4 units of a restriction endonuclease EcoRI [Takara] at 37°C for one hour, followed by the T4 DNA polymerase reaction similarly as described above and further by the SalI treatment, and a DNA fragment of 160 bp (Fig. 4 ②) was obtained by agarose gel electrophoresis. In the case of 21-Leu-hUG, the structural gene was cleaved out similarly from the pLE6527 [Japanese Patent Laid-Open Publication No. 28994/1985, deposited as E. coli XA35 (pLE6527) (FERM-BP No. 514)].

The two DNA fragments prepared as described above (Fig. 4 ① and ②) were subjected to reaction in 30 $\mu$l of a reaction mixture [20 mM Tris-HCl (pH 7.5), 10 mM MgCl$_2$, 10 mM DTT and 0.5 mM ATP] with the use of 300 units of T4 DNA ligase [Takara] at 14°C for 16 hours. After completion of the reaction, E. coli K12C600 was transformed with the reaction product to obtain a transformed strain containing the desired plasmid [hereinafter referred to as pTA1522] (Fig. 4 ③) (E. coli K12C600 (pTA1522)). Such a plasmid in 21-Leu-hUG was pTA1523, and by following the same procedure as in the case of pTA1522, a transformed strain E. coli K12C600 (pTA1523) was obtained. Similarly as in the above experimental example, plasmids were prepared from the strains obtained here (see Japanese Patent Laid-Open Publication No. 30687/1985), and the base sequences at the linking portions of the respective DNA fragments were confirmed according to the Maxam-Gilbert method [supra].

Production and recovery of hUG (21-Leu-hUG)

1) Cultivation of the transformed strain:

The transformed strain E. coli K12C600 (pTA1522) was precultured in 80 ml of an L-broth [1% bactotripton, 0.5% yeast extract, 0.5% NaCl, 0.1% glucose] containing 20 $\mu$g/ml of ampicillin. Then, the precultured broth was transferred into a synthetic medium [Biochim. Biophs. Acta 38, 470 (1960)] containing 0.64 mM potassium dihydrogen phosphate (hereinafter referred to as $KH_2PO_4$), and shaking culture was conducted at 37°C overnight. After completion of cultivation, the microorganism cells were collected and suspended in the above mentioned synthetic medium, followed further by shaking culture at 37°C for 5 hours. Also, for 21-Leu-hUG, cultivation was carried out similarly.

The culture medium had the following composition: 0.1 M Tris-HCl (pH 7.2), 80 mM NaCl, 2 mM KCl, 20 mM $NH_4Cl$, 3 mM $Na_2SO_4$, 1 mM $MgCl_2$, 0.2 mM $CaCl_2$, 2 $\mu$M $FeCl_3$, 2 $\mu$M $ZnCl_2$, 0.2% glucose, 20 $\mu$g/ml ampicillin, 40 $\mu$g/ml leucine, 40 $\mu$g/ml threonine and 10 $\mu$g/ml thiamine.

2) Recovery of hUG (21-Leu-hUG) by the osmotic shock method:

The hUG (or 21-Leu-hUG) excreted in the periplasm of the host microorganism was recovered by permitting it to be released out of the microorganism cell according to the osmotic shock method [J. Biol. Chem. 240, 3685, (1965)]. That is, the microorganism cells collected were suspended in 120 ml of a reaction mixture [20% sucrose, 30 mM Tris-HCl (pH 8.0), 1 mM EDTA] and left to stand at room temperature for 10 minutes. Then, the cells were collected, suspended in 80 ml of cold water, left to stand in an ice-bath for 10 minutes, and thereafter subjected to centrifugation, and the supernatant was recovered.

3) Quantitative determination of hUG (21-Leu-hUG):

A part of the supernatant obtained by the osmotic shock method was diluted 3-fold, and quantitative determination of hUG and 21-Leu-hUG was conducted by use thereof [the method of A. King et al (J. B. C., 257, 3053 (1982)]. More specifically, KB cells derived from human rhinopharynx epithelial cancer (ATCC No. CCL17) were subjected to mono-layer culturing in Dulbecco's modified Eagle medium (DME) (Nissui) in an 800 ml flask. The medium was removed, and the cells were peeled off with a phosphate-buffered saline solution (PBS) containing 0.05% EDTA to prepare a cell suspension. Then, the cells were washed twice with Hanks buffered saline solution (HBSS) containing 20 mM HEPES (pH 7.4). After the cells were suspended in Binding solution (DME medium, 20 mM Hepes (pH 7.4), 0.35 g/l $NaHCO_3$, 100 $\mu$g/ml streptomycin), the number of cells was calculated and adjusted to 300,000 to 400,000/0.2 ml Binding solution, and 0.2 ml each of the suspension was apportioned into a tube. 0.2 ml of each sample solution containing hUG (or 21-Leu-hUG) and $^{125}$I-mEGF (mouse EGF) at various concentrations was added into the tube, after which incubation was carried out at 37°C for one hour. The cells were washed with ice-cooled HBSS (x 3) and suspended in 10 % trichloroacetic acid (hereinafter referred to as TCA), and the cells were fixed with the use of a glass filter. After removal of TAC with acetone, counting was performed by means of a liquid scintillation counter.

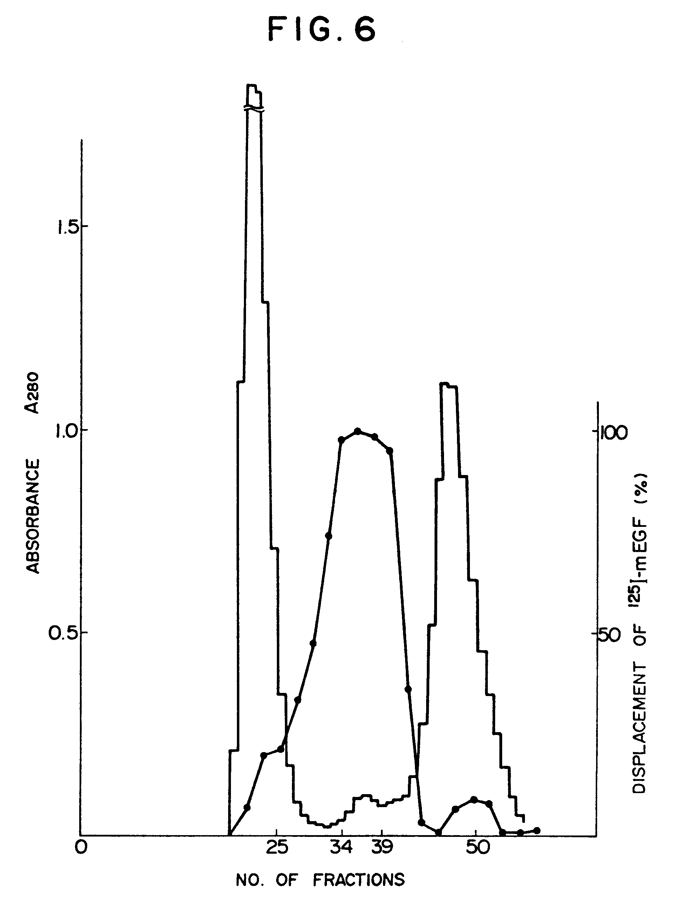

For comparison, the same experiments were conducted for the peptide fractions obtained by the cyanogen bromide treatment of mEGF (Toyobo) and $\beta$-galactosidase. Fig. 5 shows the results obtained (in which the marks ● denote mEGF and the marks ○ hUG). As is apparent from Figure 5, the peptide fraction containing hUG interferes competitively with binding between the $^{125}$I-mEGF and the KB cell EGF receptor, its dose-reaction curve being fairly identical with that of mEGF.

As the result, it was confirmed that hUG contained a peptide having EGF activity of 6.24 ng/$\mu$l as calculated on mEGF. This means that 208 $\mu$g per liter of culture (hereinafter written $\mu$g/liter-culture) was produced by excretion. Also, for 21-Leu-hUG, quantitative determination was conducted similarly to obtain the result that 185 $\mu$g/liter-culture was excreted.

13

Purification of hUG (21-Leu-hUG)

The supernatant obtained by the osmotic shock method was lyophilized into powder, which was dissolved in 6 ml of water and applied onto a Sephadex® G-50 column (diameter 7.5 cm x length 90 cm) equilibrated with 25 mM ammonium acetate (hereinafter referred to as $AcONH_4$) (pH 5.8). Elution was performed at a flow rate of 9.5 ml/fraction/14 minutes, and 34 to 39 fractions having activities were collected. The elution pattern and the results of measurement of activities (according to the above RRA method) when subjected to Sephadex® G-50 are shown in Fig. 6, in which ●—● indicate activities.

Subsequently, the fractions (34 - 39 fractions) were applied onto the DEAE Sepharose DE-52 (Whatman Co.) [diameter 1.2 cm x length 12 cm] equilibrated with 25 mM $AcONH_4$ (pH 5.8). During the elution, the buffer of $AcONH_4$ was employed at a concentration gradient of from 25 mM to 300 mM (300 ml), elution was performed at a rate of 3.12/fraction/7.6 min., and 39 to 45 fractions having activities were collected. The elution pattern and the results of measurement of activities (according to the above RRA method) are shown in Fig. 6, in which ●—● indicate activities.

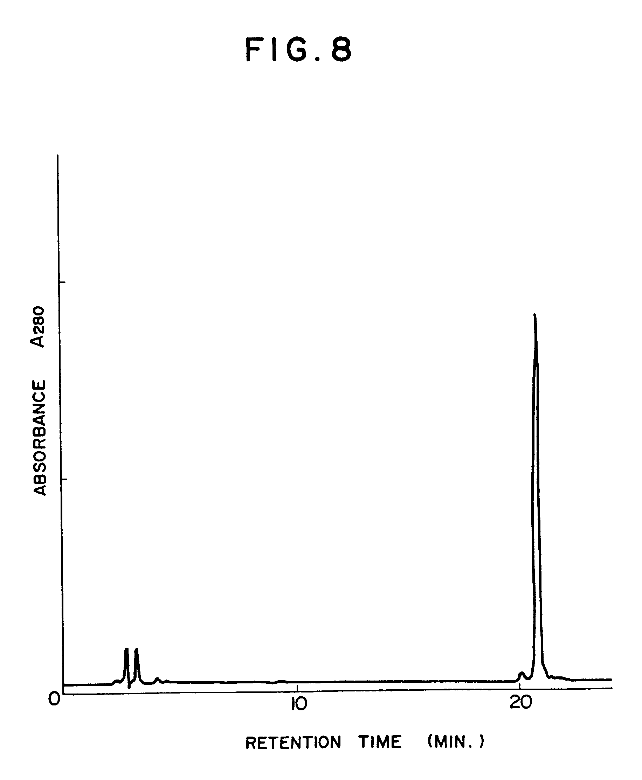

Subsequently, the fractions having activities thus obtained were purified by high performance liquid chromatography (hereinafter referred to as HPLC) [μBondapak C - 18, column: diameter 0.6 cm x length 30 cm, elution: 0.1% TFA, concentration gradient of acetonitrile from 20% to 50%, flow rate: 1 ml/min.]. The elution pattern was as shown in Fig. 8, in which a single peak ascribable to hUG was obtained at the retention time of 24 minutes. Purification was also conducted similarly for 21-Leu-hUG.

Confirmation of hUG (21-Leu-hUG)

Confirmation of hUG (21-Leu-hUG) was performed by analysis of amino acid composition, N-end analysis and C-end analysis.

Analysis of amino acid composition was carried out by hydrolyzing the fractions purified by the above HPLC with 6N HCl, drying the hydrolyzate, dissolving the dried product in 0.02 HCl and subjecting the resultant solution to an amino acid analyzer [HLC-803D OPA system (Toyo Soda)]. The results obtained were as shown in Table 1.

On the other hand, N-end analysis was conducted according to the Edman method (Biological Experimental Course 1, Chemistry of Proteins II, p. 132, edited by Society of Biochemistry of Japan, published by Tokyo Kagaku Dojin) to confirm the amino acid sequence from the N-end to the 20th amino acid.

Further, the C-end analysis was carried out by the use of carboxy peptidase Y to confirm the amino acid sequence from the C-end to the fourth amino acid.

From these results, the product excreted was confirmed to be hUG.

Also, concerning 21-Leu-hUG previously proposed by us, it was confirmed according to the same series of operations as described above.

Table 1

| Amino acid | Amino acid composition (relative ratio) | | Ref. Literature value |
|---|---|---|---|
| | Measured value | Integer | |
| Asp | 7.0 | 7 | 7 |
| Thr | 0 | 0 | 0 |
| Ser | 3.2 | 3 | 3 |
| Glu | 5.1 | 5 | 5 |
| Pro | 1.4 | 1 | 1 |
| Gly | 4.3 | 4 | 4 |
| Ala | 2.3 | 2 | 2 |
| Cys | - | - | 3 |
| Val | 2.7 | 3 | 3 |
| Met | 0.4 | 1 | 1 |
| Ile | 1.9 | 2 | 2 |
| Leu | 4.8 | 5 | 5 |
| Leu | - | - | - |
| Tyr | 4.5 | 5 | 5 |
| Phe | 0 | 0 | 0 |
| His | 2.2 | 2 | 2 |
| Trp | - | - | 2 |
| Lys | 1.7 | 2 | 2 |
| Arg | 2.9 | 3 | 3 |

In the above Table, Asp, etc. are, of course, symbols indicating amino acids such as aspartic acid as approved in this field of the art.

Comparative Example 1

In Example 1, hUG (21-Leu-hUG) was produced by the use of a plasmid having a signal peptide. As a comparative test for examination of the secretion function of the signal peptide, a plasmid pTA1502 having no signal peptide was prepared and production of hUG was attempted similarly.

In the following description, no detailed description is given relative to the restriction endonucleases, ligase reaction and preparation of DNA fragments, for which the same descriptions as in Example 1 are applicable. Also, a restriction endonuclease is denoted, for example, by AatII

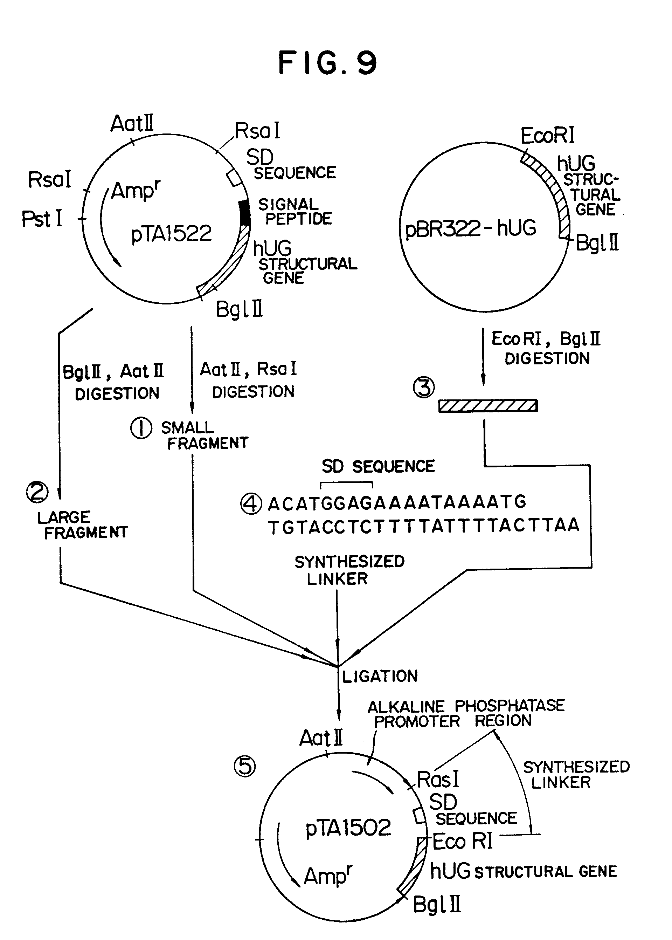

Preparation of pTA1502 (see Fig. 9)

After pTA1522 [supra] was digested with AatII and RsaI, a small fragment was obtained (in Fig. 9, ①). Similarly, after treatment of pTA 1522 with BglII and AatII, a large fragment was obtained (in Fig. 9, ②).

On the other hand, by treating pBR322-hUG with EcoRI and BglII, the structural gene of hUG (partially deficient) was obtained (in Fig. 9, ③). Also, a linker for linking the above DNA fragments ① through ③ was prepared (in the same Figure, ④). Subsequently, these fragments ① - ④ were bound according to the methods as required to obtain pTA1502 having the structural gene of hUG (in the same Figure, (4)). Production, recovery, purification, etc. of hUG were all performed in the same manner as in the above Example 1.

Quantitative determination of hUG was performed for the three fractions described below when production of protein was conducted by the use of pTA1522 and pTA1502. The results were as shown in Table 2.

(a) The fraction prepared by destroying the microorganism cells after expression of the desired protein.

(b) The supernatant fraction obtained by subjecting the host microorganism to the osmotic shock method after expression of the desired protein.

(c) The fraction obtained by destroying the microorganism cells after the osmotic shock method.

Table 2

| Plasmid employed | (a) | (b) | (c) |
|---|---|---|---|
| pTA1522 (having signal peptide) | 211 $\mu$g/liter-culture | 205 $\mu$g/liter-culture | 14.4 $\mu$g/liter-culture |
| pTA1502 (having no signal peptide) | 4.2 $\mu$g/liter-culture | 0 | 0 |

Example 2

In the above Example 1, the process of the present invention was practiced with the use of pTA1529. In this Example 4, hUG was produced similarly as described above by constructing a plasmid pTA1632 having the secretion function similarly as pTA1529 (the signal peptide of pTA1529 derived from alkaline phosphatase) and containing the structural gene of hUG (the signal peptide of pTA1632 derived from $\beta$-lactamase).

Construction of pTA1632

1) Synthesis of the signal peptide of $\beta$-lactamase (hereinafter referred to as bla signal):
The DNA gene coding for $\beta$-lactamase having the sequence as shown below was prepared according to a conventional method for synthesis of oligonucleotide. The amino acid sequence of bla used followed that of Proc. Natl. Acad. Sci., 75, 3737 - 3741 (1978).

```
EcoRI         Met Ser Ile Gln His Phe
    ↑
   ┌AATTC ATG TCT ATC CAG CAT TTC
   └─────┐
     TTAAG TAC AGA TAG GTC GTA AAG
        ↓

   Arg Val Ala Leu Ile Pro Phe Phe
   CGT GTT GCT CTG ATC CCG TTC TTC
   GCA CAA CGA GAC TAG GGC AAG AAG

   Ala Ala Phe Cys Leu Pro Val Phe
   GCT GCT TTC TGC CTG CCG GTT TTC
   CGA CGA AAG ACG GAC GGC CAA AAG

   Ala↑
   GCC┊GGC A̲AGCTT
   CGG┊CCG TTCGAA
      ↓          ↓
   Nae I     Hind III
```

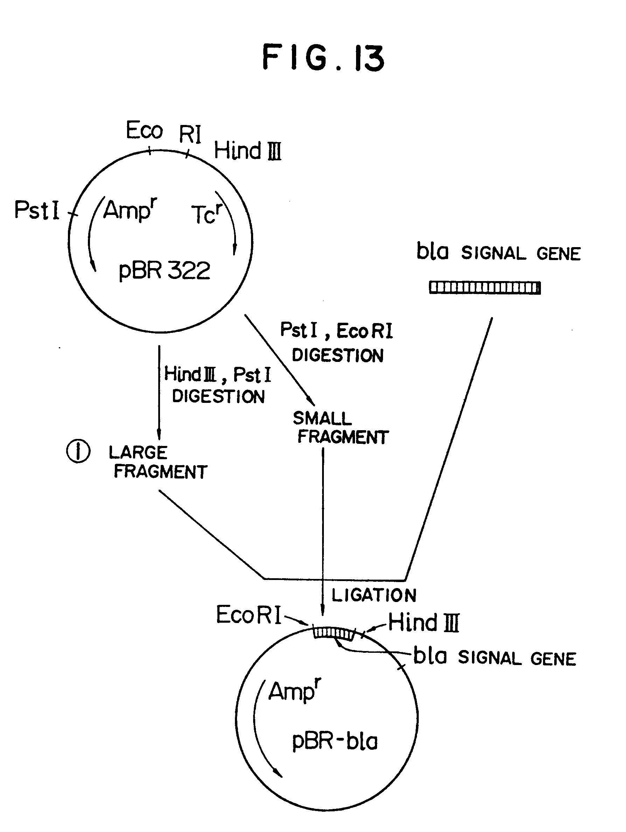

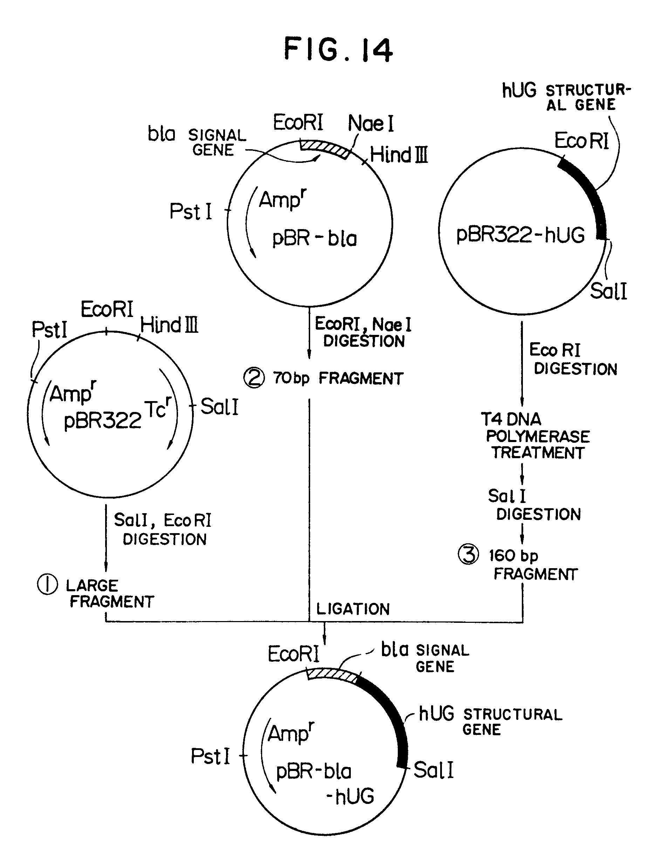

In the above sequence, <----> denotes the restriction endonuclease cleavage site.
2) Cloning of the bla signal into pBR322 (see Fig. 13):
The gene of the bla signal synthesized above was constructed following the procedure shown below. After digesting pBR322 with HindIII and PstI, a large DNA fragment was recovered (Fig. 13 ①). Also, after digesting separately pBR322 with PstI and EcoRI, a small DNA fragment was recovered (Fig. 13 ② ). These two DNA fragments were ligated with the bla signal DNA fragment to obtain a plasmid pBR-bla (Fig.13 ③).
3) Linking of hUG gene (Fig. 14):
Following the procedure shown below, the hUG structural gene was inserted into the above plasmid (pBRbla).

After digesting pBR322 with SalI and EcoRI, a large DNA fragment was recovered ( ① ). After digesting the above pBR-bla with EcoRI and NaeI, a DNA fragment of about 70 bp was recovered (②). On the other hand, the pBR322-hUG (supra) was subjected to EcoRI digestion, then to the T4 DNA polymerase treatment and further to SalI digestion to obtain a DNA fragment of about 160 bp (③). Finally, the above DNA fragments (①, ② and ③) were linked to obtain a plasmid pBR-bla-hUG having the structural gene of hUG linked immediately after the bla signal.

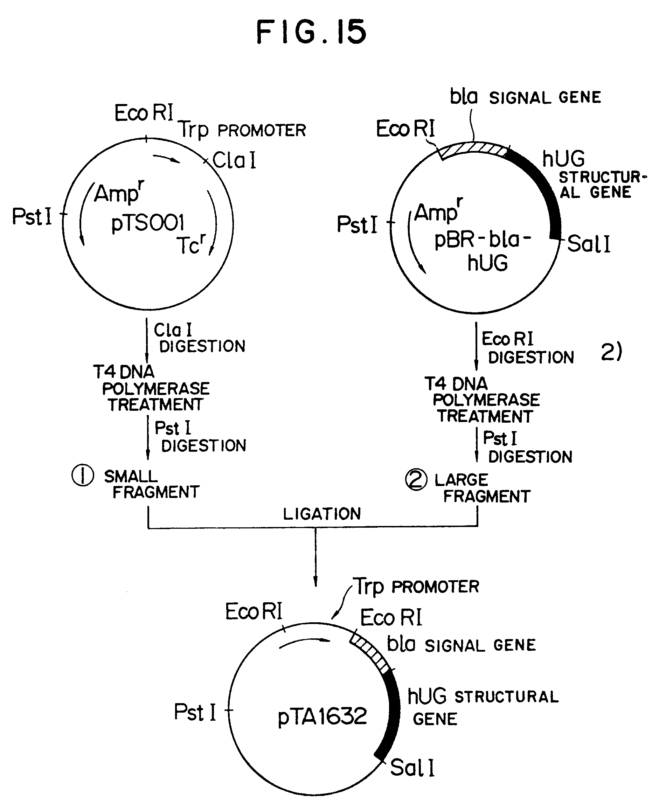

4) Linking to tryptophan promoter (Trp) (Fig.15):

Following the procedure shown below, a plasmid pTA1632 with hUG being placed under the control of triptophan promoter was constructed.

The pTS001 (prepared by insertion of Trp promoter into pBR322 at the EcoRI recognition site in the direction of the tetracycline resistant gene, followed by removal of the attenuator portion) was digested with ClaI,then treated with T4 DNA polymerase and further digested with PstI to recover a small DNA fragment ( ① ). Also, the above pBR-bla-hUG was digested with EcoRI, treated with T4 DNA polymerase and further digested with PstI to recover a large fragment (②). Finally, the above two (① and ②) DNA were linked to obtain pTA1632. The method of obtaining pTA1632 and determination of its base sequence were performed similarly as in the foregoing Examples.

Production and recovery of hUG

Production and recovery of hUG were conducted similarly as in the above Example 1. As a result, 180 μg of hUG was found to be produced per one liter of culture.

Example 3

Preparation of plasmid

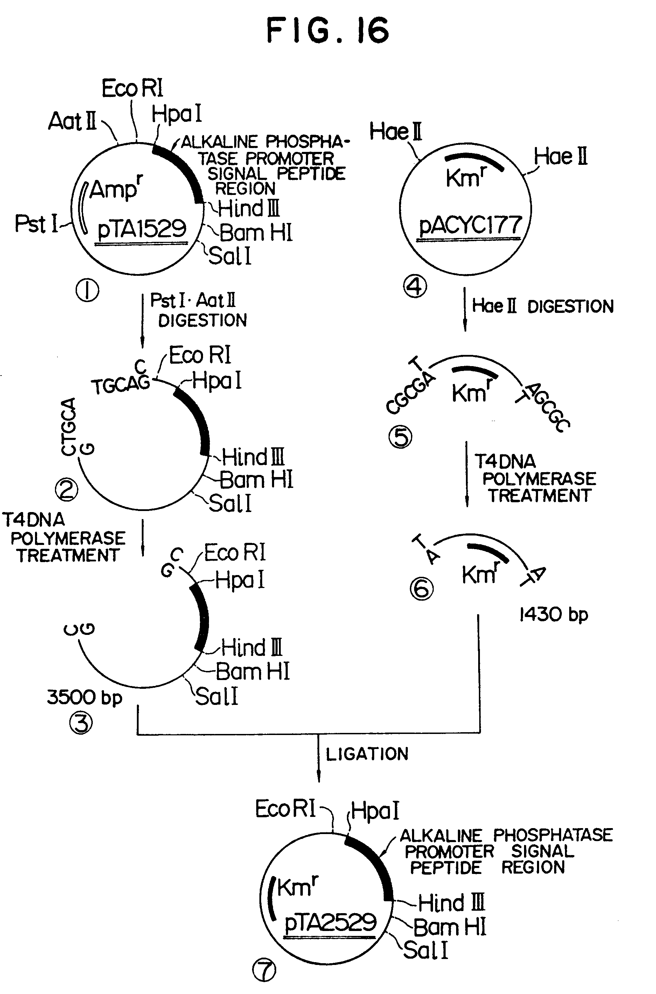

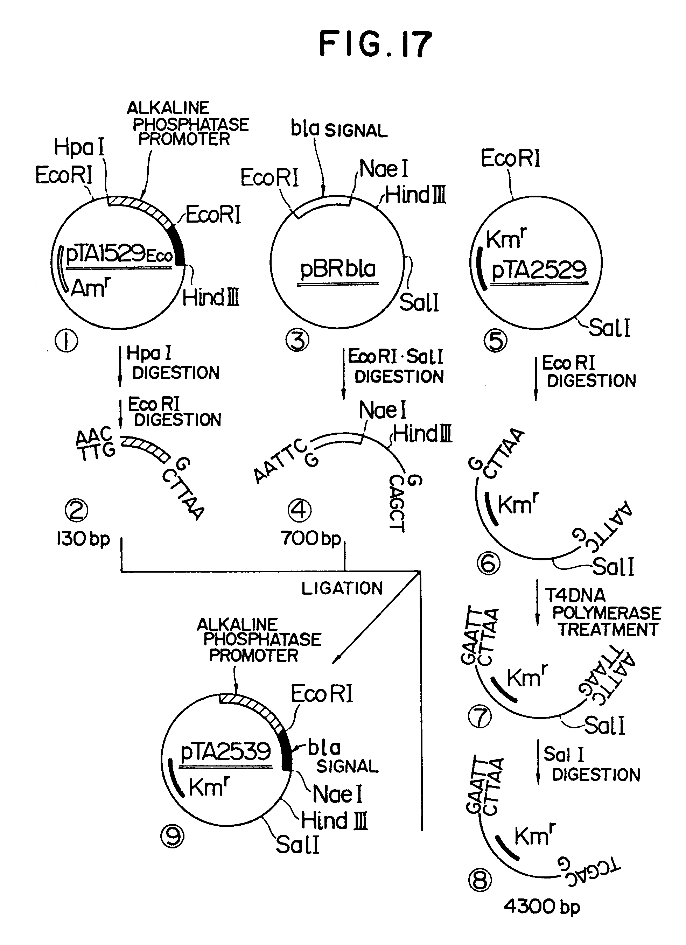

Preparation of a plasmid having the secretion function and having no gene coding for secretable protein derived from the plasmid was carried out as follows. The steps are as shown in Figs. 16 and 17.

1) Preparation of pTA2529:

(a) After treatment of 5 μg of the plasmid 1529 previously proposed by us (Fig. 16 ①) in 50 ml of a reaction mixture [50 mM Tris-hydrochloride buffer (hereinafter abbreviated Tris-HCl), 50 mM sodium chloride (hereinafter abbreviated NaCl), 10 mM magnesium chloride (hereinafter abbreviated MgCl₂)] with 4 units of a restriction endonuclease PstI (Takara Co.) (hereinafter abbreviated PstI) (1 μl) and 4 units of a restriction endonuclease AatII (Toyobo Co.) (hereinafter referred to as AatII) at 37°C for one hour, the product was extracted with phenol and subjected to ethanol precipitation to recover 2 in Fig. 16. The precipitate was treated in 50 μl of a reaction mixture [67 mM Tris-HCl, 6.7 mM MgCl₂, 6.7 mM ethylenediaminetetraacetic acid (hereinafter abbreviated EDTA), 16.6 mM ammonium sulfate (hereinafter abbreviated (NH₄)₂SO₄), 10 mM mercaptoethanol, 0.66 mM dATP, 0.66 mM TTP, 0.66 mM dCTP and 0.66 mM dGTP] with one unit of T4-DNA polymerase (BRL Co.) at 37°C for 15 minutes, after which agarose electrophoresis was carried out, to recover the desired fragment (Fig. 16 ③).

(b) In 50 μl of a reaction mixture [500 mM Tris-HCl (pH 7.4), 10 mM MgCl₂, 50 mM NaCl], 5 μg of pACYC177 (Journal of Bacteriology, 134, No. 3, 1141 - 1156 (1978)) (Fig. 16 ④) was treated with 4 units of a restriction endonuclease HaeII (Takara Co.) (hereinafter referred to as HaeII) at 37°C for 60 minutes. The product was extracted with phenol and precipitated with ethanol to recover ⑤ in Fig. 16. The precipitate was treated with one unit of T4-DNA polymerase (BRL Co.) similarly as described above. By agarose gel electrophoresis, a fragment (Fig. 16 ⑥ ) was recovered.

The two DNA fragments obtained in the above operations (a) and (b) (Fig. 16 ③ and ⑥ ) were allowed to react in 30 μl of a reaction mixture [20 mM Tris-HCl (pH 7.5), 10 mM MgCl₂, 10 mM DTT, 0.5 mM ATP] with 30 units of T₄ DNA ligase (Takara) at 14°C for 16 hours. After completion of the reaction, E. coli K12C600 (FERM-BP No. 115) was transformed with the reaction product to obtain a transformed strain containing the desired plasmid (pTA529).

17

2) Preparation of pTA2539:

(a) After 5 $\mu$g of the pTA1529Eco (Fig. 17 ① ) prepared according to the method described in the specification of Japanese Patent Laid-Open Publication No. 149087/1986, was treated in 50 $\mu$l of a reaction mixture [10 mM Tris-HCl (pH 8), 10 mM MgCl$_2$, 100 mM.NaCl] with 4 units of a restriction endonuclease HpaI (Takara) (hereinafter abbreviated HpaI), the product was extracted with phenol and subjected to ethanol precipitation. The resultant precipitate was treated in 50 $\mu$l of a reaction mixture [100 mM Tris-HCl (pH 7.5), 50 mM NaCl, 10 mM MgCl$_2$] with 1 $\mu$l of 4 units of EcoRI (Takara) (hereinafter referred to as EcoRI) at 37°C for one hour, which treatment was followed by acrylamide gel electrophoresis, to obtain the desired fragment (Fig. 17 ② ).
(b) After 5 $\mu$g of the pBR-bla prepared by us (Fig. 17 ③ ) was treated similarly in a reaction mixture [10 mM Tris-HCl (pH 8), 10 mM MgCl$_2$, 150 mM NaCl] with 1 $\mu$l of 4 units of EcoRI and 1 $\mu$l of 4 units of a restriction endonuclease SalI (Takara Co.) (hereinafter referred to as SalI) at 37°C for one hour, agarose electrophoresis was performed to obtain the desired fragment (Fig. 17 ④).
(c) After 5 $\mu$g of the pTA2529 as prepared above (Fig. 17 ⑤ ) was treated in 50 $\mu$l of a reaction mixture [100 mM Tris-HCl (pH 7.5), 50 mM NaCl, 10 mM MgCl$_2$] with 1 $\mu$l of 4 units of EcoRI at 37°C for one hour, the product was extracted with phenol and subjected to ethanol precipitation. The resultant precipitate ⑥ (Fig. 17) was treated similarly as described above with 1 $\mu$l of one unit of T$_4$ DNA polymerase (BRL Co.). Further, after treatment in 50 $\mu$l of a reaction mixture [10 mM Tris-HCl (pH 8), 150 mM NaCl, 10 mM MgCl$_2$] with 1 $\mu$l of 4 units of SalI at 37°C for one hour, the desired fragment (Fig. 17 ⑧) was recovered by agarose gel electrophoresis.
The three DNA fragments obtained in the above operations (a) to (c) were allowed to react in 30 $\mu$l of a reaction mixture [20 mM Tris-HCl (pH 7.5), 10 mM MgCl$_2$, 10 mM DTT, 0.5 mM ATP] with 300 units of T$_4$ DNA ligase (Takara Co.) at 14°C for 16 hours. After completion of the reaction, E. coli YK537 [deposited as E. coli K12YK537 (FERM-BP No. 822)] was transformed with the reaction product to obtain the desired transformed strain. From the strain obtained here, a plasmid was prepared (see Japanese Patent Laid-Open Publication No. 30687/1985), and the base sequences of the linking portions of the respective fragments were confirmed by the Maxam-Gilbert method [Proc. Natl. Acad. Sci. USA, 74, 560 (1977)].

Production of protein

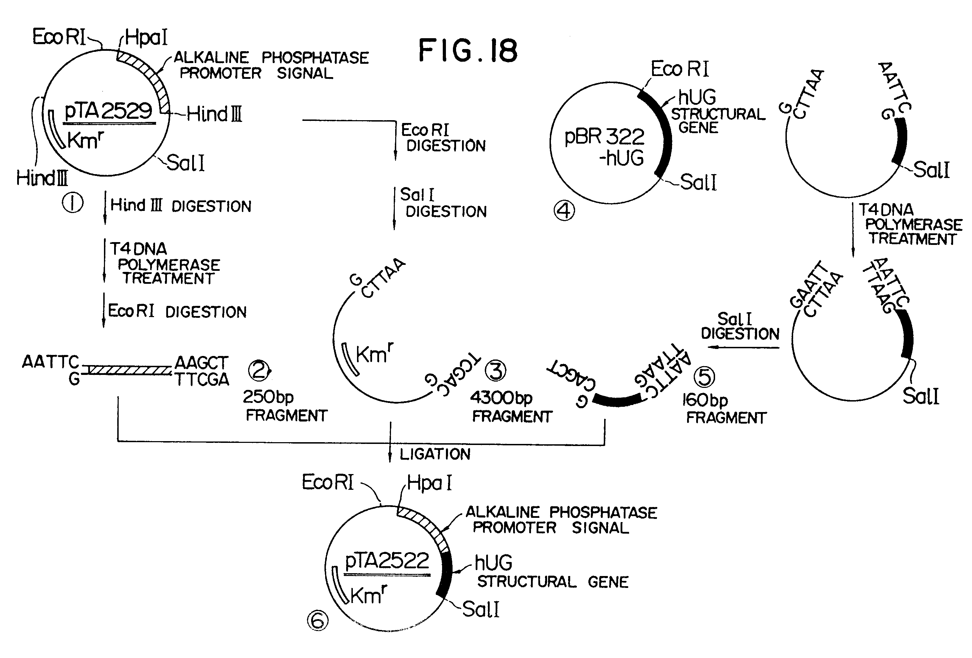

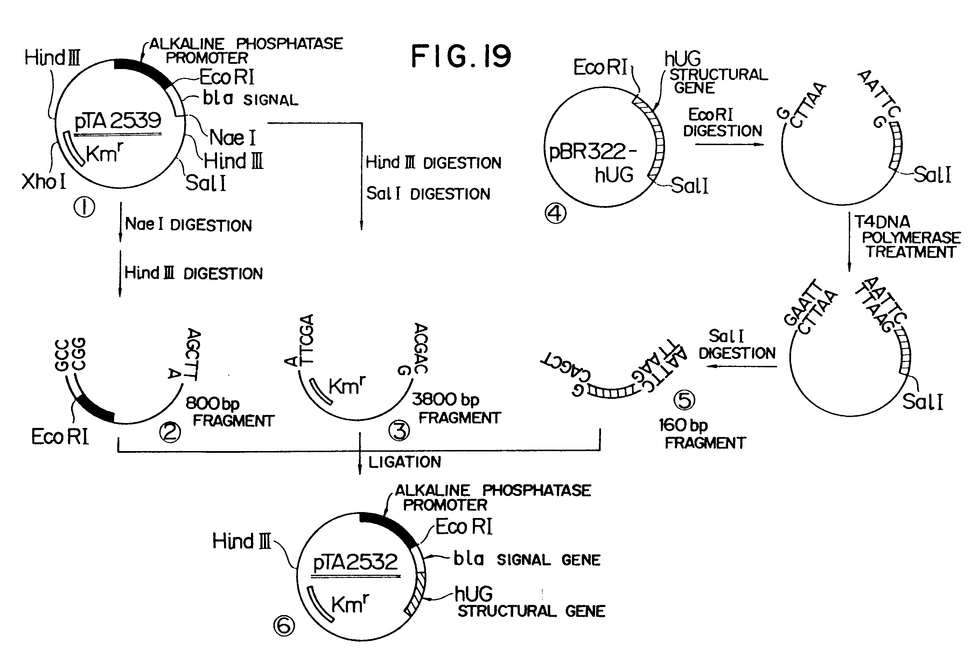

Human urogastrone (hUG/EGF) was incorporated in the above plasmid of the present invention, and its production and recovery were performed following the genetic engineering technique. The steps are as shown in Figs. 18 and 19.

Synthesis of hUG structural gene

Synthesis of the hUG structural gene was carried out similarly as in the foregoing Examples.

Preparation of plasmid containing hUG gene

1) Preparation of transformed strain [E. coli K12YK537 (pTA2522):

(a) Hydrolysis of 5 $\mu$g of pTA2529 (Fig. 18 ①) was carried out in 50 $\mu$l of a buffer [10 mM Tris-HCl (pH 7.5), 10 mM MgCl$_2$, 50 mM NaCl] with 4 units of a restriction endonuclease Hind III (Takara) (hereinafter referred to as Hind III) at 37°C for one hour. Then, the precipitate obtained by ethanol precipitation was treated in 30 $\mu$l of a reaction mixture [67 mM Tris-HCl (pH 8.8), 16.6 mM (NH$_4$)$_2$SO$_4$, 6.7 mM MgCl$_2$, 10 mM 2-mercaptoethanol, 6.7 mM EDTA, each 0.66 mM of dATP, dCTP, dGTP, TTP] with one unit of T$_4$ DNA polymerase (supra) at 37°C for 15 minutes. Then, after ethanol precipitation, the resultant precipitate was treated in 50 $\mu$l of a reaction mixture [100 mM Tris-HCl (pH 7.5), 50 mM NaCl, 10 mM MgCl$_2$] with 1 $\mu$l of 4 units of EcoRI at 37°C for one hour, after which polyacrylamide gel electrophoresis was carried out, to recover the desired DNA fragment of 250 bp (Fig. 18 ②).
(b) Treatment of 5 $\mu$g of the pTA2529 (Fig. 18 ①) was carried out in 50 $\mu$l of a reaction mixture [100 mM Tris-HCl, 50 mM NaCl, 10 mM MgCl$_2$] with 1 $\mu$l of 4 units of EcoRI at 37°C for one hour. Then, after ethanol precipitation, the resultant precipitate was hydrolyzed in 50 $\mu$l of a reaction mixture [6 mM Tris-HCl (pH 8), 6 mM MgCl$_2$, 150 mM NaCl] with 4 units of a restriction endonuclease SalI (Takara) (hereinafter referred to as SalI) at 37°C for one hour. After completion of the reaction, a DNA fragment of 4,300 bp (Fig. 18 ③) was obtained by agarose gel electrophoresis.

18

After 5 $\mu$g of the plasmid pBR322-hUG [the pBR322 (Fig. 18 ④), deposited as E. coli K12C600 (pBR322) (FERM-BP No. 235), digested with EcoRI and SalI, having the fragment of the hUG structural gene as synthesized above digested with EcoRI and SalI incorporated therein] was hydrolyzed in 50 $\mu$l of a reaction mixture [100 mM Tris-HCl (pH 7.5), 50 mM NaCl, 50 mM MgCl$_2$] with 4 units of EcoRI (Takara) at 37°C for one hour, the T$_4$ DNA polymerase reaction was carried out similarly as described above, which procedure was followed further by the SalI treatment, and thereafter a DNA fragment of 160 bp was obtained by agarose gel electrophoresis (Fig. 18 ⑤).

The three DNA fragments as prepared above (Fig. 18 ②, ③ and ⑤) were allowed to react in 30 $\mu$l of a reaction mixture [20 mM Tris-HCl (pH 7.5), 10 mM MgCl$_2$, 10 mM DTT, 0.5 mM ATP] with the use of 300 units of T$_4$ DNA ligase (Takara) at 14°C for 16 hours. After completion of the reaction, E. coli YK537 [deposited as E. coli K12YK537 (FERM-BP No. 822)] was transformed with the reaction product to obtain a transformed strain [E. coli K12YK537] containing the desired plasmid (hereinafter referred to as pTA2522) (Fig. 18 ⑥).

2) Preparation of transformed strain [E. coli K12YK537 (pTA2532)]:

(a) Treatment of 5 $\mu$g of the pTA2539 (Fig. 19 ①) was carried out in 50 $\mu$l of a reaction mixture [6 mM Tris-HCl (pH 8), 20 mM NaCl, 6 mM MgCl$_2$] with 4 units of restriction endonuclease NaeI (N.E.B. Co.) (hereinafter referred to as NaeI) at 37°C for one hour. Then, the product was extracted with phenol and recovered by ethanol precipitation. This was treated in 50 $\mu$l of a reaction mixture [10 mM Tris-HCl (pH 7.5), 10 mM MgCl$_2$, 50 mM NaCl] with 4 units of Hind III at 37°C for one hour. After completion of the reaction, by the agarose gel electrophoresis, a DNA fragment of 800 bp was obtained (Fig. 19 ②).

(b) Treatment of 5 $\mu$g of the pTA2539 (Fig. 19 ①) was carried out in 50 $\mu$l of a reaction mixture [10 mM Tris-HCl (pH 7.5), 10 mM MgCl$_2$, 50 mM NaCl] with 1 $\mu$l of 4 units of Hind III at 37°C for one hour, which treatment was followed by ethanol precipitation. The resultant precipitate was treated similarly as above with 1 $\mu$l of 4 units of a restriction endonuclease SalI (Takara) at 37°C for one hour, and then a DNA fragment of 3,800 bp was obtained by agarose gel electrophoresis (Fig. 19 ③).

(c) After 5 $\mu$g of the pBR322-hUG (Fig. 19 ④) (the pBR322 digested with EcoRI and SalI, having the fragment of the hUG structural gene as synthesized above digested with EcoRI and SalI incorporated therein] was hydrolyzed in 50 $\mu$l of a reaction mixture [100 mM Tris-HCl (pH 7.5), 50 mM NaCl, 50 mM MgCl$_2$] with 4 units of a restriction endonuclease EcoRI (Takara) at 37°C for one hour, the T$_4$ DNA polymerase reaction was carried out similarly as described above, which reaction was followed further by the SalI treatment, and thereafter a DNA fragment of 160 bp (Fig. 19 ⑤) was obtained by agarose gel electrophoresis.

The three DNA fragments prepared in the above operations (a) to (c) (Fig. 19 ②, ③ and ⑤) were allowed to react in 30 $\mu$l of a reaction mixture [20 mM Tris-HCl (pH 7.5), 10 mM DTT, 0.5 mM ATP] with the use of 300 units of T$_4$ DNA ligase (Takara Co.) at 14°C for 16 hours. After completion of the reaction, the YK537 was transformed similarly as described above to obtain a transformed strain [E. coli K12YK537 (pTA2532)] containing the desired plasmid [hereinafter written as pTA2532] (Fig. 19 ⑥). Similarly as in the above Examples, from the strain obtained here, a plasmid was prepared (see Japanese Patent Laid-Open Publication No. 30687/1985) and the base sequences of the linking portions of the respective DNA fragments were confirmed according to the Maxam-Gilbert method (supra).

Production, recovery and quantitative determination of hUG were conducted according to the same methods as in the foregoing Examples.

For the purpose of comparison of the excreted amounts between the above transformed strain [E. coli K12YK537 (pTA2522) and E. coli K12YK537 (pTA2532)], the above experiment was performed with YK537 transformed similarly by use of pTA1522 and pTA1532 (Fig. 14, pBR-bla-hUG) in which Km$^r$ of the plasmid was changed to Amp$^r$ coding for excretable proteins. The amounts of hUG after 2 hours' cultivation were as shown in Table 4. The units ($\mu$g/L) indicate the amount of hUG excreted ($\mu$g) in one liter of the culture broth.

## Table 4

| Transformed strain | | Amount secreted (μg/L) |
|---|---|---|
| E. coli K12YK537 | (pTA2522) | 1511 |
| " | (pTA2532) | 1614 |
| " | (pTA1522) | 1200 |
| " | (pTA1532) | 1106 |

From the above results, the transformed strains obtained by using the plasmid in which no gene coding for the excretable proteins derived from the plasmid exists [E. coli K12YK537(pTA2522) and E. coli K12YK537 (pTA 2532)] were found to be increased in amounts of hUG excreted by 350 to 500 μg per one liter of the culture broth as compared with the transformed strains in which the gene coding for the excretable proteins derived from the plasmid exists [E. coli K12YK537 (pTA 1532) and E. coli K12YK537-(pTA1632)].

Purification and confirmation of hUG were also practiced similarly as in the foregoing Examples by conducting amino acid analysis, N- and C-end analyses, whereby the hUG excreted was confirmed to be hUG.

Bacteriological properties of the related microorganisms and deposition numbers:

The bacteriological properties and the deposition numbers of the microorganisms disclosed in the present invention are as follows.

Representation of microorganism          Deposition number*

(1) E. coli K12C600                       FERM-BP No. 115

(2) E. coli K12C600 (pYK283)              FERM-BP No. 556

(3) E. coli K12C600 (pBR322)              FERM-BP No. 235

(4) E. coli K12YK537                      FERM-P No. 7941, which is now FERM-BP No. 822

(5) E. coli XA35 (pLE6527)                FERM-BP No. 514

(6) E. coli K12C600 (pIF202)              FERM-P No. 7188

Date of deposition:

(1) June 9, 1981        (2) April 30, 1983 (3) June 9, 1981

(4) November 14, 1984 (5) July 2, 1983    (6) August 2, 1983

   * Deposition number at Fermentation Research Institute, Agency of Industrial Science and Technology, Ministry of International Trade and Industry, the Japanese Government, 1-3, Higashi 1-Chome, Yatabe-Machi, Tsukuba-Gun, Ibaraki-Ken, 305, Japan.

Strains (1)-(5) are on deposit under the BUDAPEST TREATY ON THE INTERNATIONAL RECOGNITION OF THE DEPOSIT OF MICROORGANISMS FOR THE PURPOSES OF PATENT PROCEDURE, and will be maintained and released under the TREATY in compliance with Rule 11.3 and Rule 9 of the TREATY.

Bacteriological properties

(1) E. coli K12C600:

This microorganism is a Gram-negative rod bacterium, having the general properties of the genus Escherichia such as formation of no spores and facultative anaerobic characteristic, otherwise containing no F factor, and being deficient in the function of the suppressor gene E and defective in the recBC gene coding for the nuclease participating in recombination of the gene. As the nutrient demand, threonine and leucine are required for proliferation on its minimum medium. Also, in taxonomy, it belongs to the family of enteric bacteria and the genus Escherichia. The literatures concerning this microorganism are shown below.
(a) Genetics, 39, 440 (1954)
(b) Nature, 217, 1110 (1968)

(2) E. coli K12C600 (pYK283):

This microorganism is a Gram-negative rod bacterium, having the general properties of the genus Escherichia such as formation of no spores and facultative anaerobic characteristic, otherwise containing no F factor, and being deficient in the function of the suppressor gene E and defective in the recBC gene coding for the nuclease participating in recombination of the gene. As the nutrient demand, threonine and leucine are required for proliferation on its minimum medium. It contains the plasmid pYK 283 constituted of the replication initiation region derived from the promoter-operator region pACYC177 of the pho A gene and the bla gene of pBR322, exhibiting resistance to ampicillin. Also, in taxonomy, it belongs to the family of enteric bacteria and the genus Escherichia.
Except for the properties derived from the plasmid pYK 283, the bacteriological properties of this microorganism are the same as those of the parent strain E. coli K12C600.

(3) E. coli K12C600 (pBR322):

This microorganism is a Gram-negative rod bacterium, having the general properties of the genus Escherichia such as formation of no spores and facultative anaerobic characteristic, otherwise containing no F factor, and being deficient in the function of the suppressor gene E and defective in the recBC gene coding for the nuclease participating in recombination of the gene. As the nutrient demand, threonine and leucine are required for proliferation on its minimum medium. It also contains a drug resistant pBR 322. As to the plasmid pBR322, reference may be made to Gene, 2, 95 (1977), and as to E. coli, to the above Nature. Except for the properties derived from the plasmid pBR322, the bacteriological properties of E. coli K12C600 (pBR322) are the same as those of the parent strain.

(4) E. coli K12YK537:

The E. coli K12YK537 was obtained by further modifying the E. coli K12RR1 [Gene, 2, 95 (1977), Biochem, Biophs. Acta., 655, 243 (1981)] derived from the E. coli K12 strain which was a known strain [Microbiological Reviews, 44, 1 - 56 (1980)], exhibiting the properties as shown below, other properties being the same as those of K12RR1:
[rec AI, Pho A8, pro⁺].
That is, the YK537 is a Gram-negative rod bacterium, having the general properties of the genus Escherichia such as formation of no spores and facultative anaerobic characteristic, and otherwise containing no F factor, and being deficient in the function of the suppressor gene E and defective in the recBC gene coding for the nuclease participating in recombination of the gene. As the nutrient demand, threonine and leucine are required for proliferation on its minimum medium. This strain is deficient in expression of alkali phosphatase from chromosome possessed by ordinary E. coli strains [deposited as E. coli K12YK537 (FERM-BP No. 822)].

(5) E. coli XA35 (pLE6527)

E. coli XA35 (pLE6527) is a transformant of E. coli XA35 by means of a plasmid pLE6527. The host strain E. coli XA35 is a derivative of known E. coli K12 [See Microbiological Reviews, 44, 1-56 (1980)] having the same characteristics as the E. coli K12 has except for the following:

[$Sm^r$, $Lac^-$ ($i_3^-$, z)]

The transformant E. coli XA35 (pLE6527) has the same characteristics as the host E. coli XA35 has except for the following:

[$Amp^r$, $Lac^+$]

The plasmid pLE6527 is such a hybrid that a DNA fragment comprising a part of lactose operon derived from λ plac 5DNA and a gene of artificial human urogastrone [$^{21}$Met → $^{21}$Leu] have been inserted into pBR322 plasmid.

(6) E. coli K12C600 (pIF202)

E. coli K12C600 (pIF202) is a transformant of E. coli K12C600, which is referred to above, by means of a plasmid pIF202. The transformant E. coli K12C600 (pIF202) has the same characteristics as the host E. coli K12C has except for those due to the plasmid pIF202. The transformant E. coli K12C600 (pIF202) has a genotype of $Amp^r$ and of $Tc^s$ at 30°C but $Tc^r$ at 42°C. The plasmid pIF202 is such a hybrid that a $CI-P_R-P_L$ region derived from λCI857S7DNA and an α-interferon gene derived from human lenkocyte have been inserted into pBR322 plasmid.

The plasmid pIF202 can be constructed from E. coli K12C600 (pIF101), which is on deposit at the above-captioned Japanese depository under the above-captioned BUDAPEST TREATY with an accession number FERM-BP No. 595 dated August 2, 1983, according to a procedure as set forth in Japanese Patent Laid-Open Publication No. 66992/1985.

**Claims**

1. A recombinant DNA vector comprising a gene encoding a natural signal peptide derived from Escherichia coli and a gene encoding a human epidermal growth factor or a 21-Leu epidermal growth factor, having the amino-acid sequence of Fig 3 minus the N-terminal methionine, the former gene being linked to the latter gene without any intervening nucleotide(s) therebetween said gene product being cleaved by a signal peptidase present in the Escherichia coli cells thus permitting secretion of mature epidermal growth factor out of the cell.

2. The recombinant DNA vector of Claim 1, wherein said recombinant DNA vector comprises a gene coding for a promoter, a gene coding for the Shine-Dalgarno sequence, said gene encoding the natural signal peptide, and said gene encoding the epidermal growth factor, wherein said vector contains no other gene encoding an excretable protein.

3. The recombinant DNA vector of Claim 1, wherein said gene encoding a natural signal peptide comprises at least a part of an artificially created restriction endonuclease recognition site.

4. The recombinant DNA vector of Claim 3, wherein the upstream part of the restriction endonuclease cleavage site of said restriction endonuclease recognition site is situated other than downstream of the boundary between said gene encoding a natural signal peptide and said gene encoding the epidermal growth factor.

5. The recombinant DNA vector of Claim 4, wherein said restriction endonuclease cleavage site is situated at the boundary between said gene encoding a natural signal peptide and said gene encoding the epidermal growth factor.

6. The recombinant DNA vector of Claim 3, wherein said gene encoding a natural signal peptide comprises two moieties: a first moiety situated upstream of the restriction endonuclease cleavage site of said restriction endonuclease recognition site and being derived from Escherichia coli; and a second moiety situated downstream of said restriction endonuclease cleavage site and being artificially synthesized.

22

7. The recombinant DNA vector of Claim 3, wherein said gene encoding a natural signal peptide is derived from alkaline phosphatase, and said restriction endonuclease recognition site is the Hind III recognition site situated at the boundary between said gene encoding a natural signal peptide and said gene encoding the epidermal growth factor.

8. The recombinant DNA vector of Claim 3, wherein said gene encoding a natural signal peptide is derived from β-lactamase, and said restriction endonuclease recognition site is the Nae I recognition site situated at the boundary between said gene encoding a natural signal peptide and said gene encoding the epidermal growth factor.

9. A process for producing a human epidermal growth factor or a 21-Leu epidermal growth factor having the amino-acid sequence of Fig 3 minus the N-terminal methionine, comprising:
(a) culturing Escherichia coli cells which have been transformed with a recombinant DNA vector which comprises a gene encoding a natural signal peptide derived from Escherichia coli and a gene encoding the epidermal growth factor, the former gene being linked to the latter gene without any intervening nucleotide(s) therebetween;
(b) allowing a signal peptidase present in the Escherichia coli cells to cleave said gene product to permit secretion of the mature epidermal growth factor out of the cell; and
(c) recovering said epidermal growth factor in mature form.

10. The process of Claim 9, wherein said recombinant DNA vector comprises a gene coding for a promoter, a gene coding for the Shine-Dalgarno sequence, said gene encoding the natural signal peptide, and said gene encoding the epidermal growth factor, wherein said vector contains no other gene encoding an excretable protein.

11. The process of Claim 9, wherein said gene encoding a natural signal peptide comprises at least part of an artificially created restriction endonuclease recognition site.

12. The process of Claim 11., wherein the upstream part of the restriction endonuclease cleavage site of said restriction endonuclease recognition site is situated other than downstream of the boundary between said gene encoding a natural signal peptide and said gene encoding the epidermal growth factor.

13. The process of Claim 12., wherein said restriction endonuclease cleavage site is situated at the boundary between said gene encoding a natural signal peptide and said gene encoding the epidermal growth factor.

14. The process of Claim 11., wherein said gene encoding a natural signal peptide comprises two moieties: a first moiety situated upstream of the restriction endonuclease cleavage site of said restriction endonuclease recognition site and being derived from Escherichia coli; and a second moiety situated downstream of said restriction endonuclease cleavage site and being artificially synthesized.

15. The process of Claim 11., wherein said gene encoding a natural signal peptide is derived from alkaline phosphatase, and said restriction endonuclease recognition site is the Hind III recognition site situated at the boundary between said gene encoding a natural signal peptide and said gene encoding the epidermal growth factor.

16. The process of Claim 11., wherein said gene encoding a natural signal peptide is derived from β-lactamase, and said restriction endonuclease recognition site is the Nae I recognition site situated at the boundary between said gene encoding a natural signal peptide and said gene encoding the epidermal growth factor.

17. A cell capable of producing a human epidermal growth factor or a 21-Leu epidermal growth factor, having the amino-acid sequence of Fig 3 minus the N-terminal methionine, wherein said cell is transformed with a recombinant DNA vector which comprises a gene encoding a natural signal peptide derived from Escherichia coli and a gene encoding the epidermal growth factor, the former gene being linked to the latter gene without any intervening nucleotide(s) therebetween said gene product being cleaved by a signal peptidase present in the Escherichia coli cells thus permitting secretion of mature epidermal growth factor out of the cell.

**18.** The cell of Claim 17, wherein said recombinant DNA vector comprises a gene coding for a promoter, a gene coding for the Shine-Dalgarno sequence, said gene encoding the natural signal peptide, and said gene encoding the epidermal growth factor, wherein said vector contains no other gene encoding an excretable protein.

**19.** The cell of Claim 17, wherein said gene encoding a natural signal peptide comprises at least a part of an artificially created restriction endonuclease recognition site.

**20.** The cell of Claim 19, wherein the upstream part of the restriction endonuclease cleavage site of said restriction endonuclease recognition site is situated other than downstream of the boundary between said gene encoding a natural signal peptide and said gene encoding the epidermal growth factor.

**21.** The cell of Claim 20, wherein said restriction endonuclease cleavage site is situated at the boundary between said gene encoding a natural signal peptide and said gene encoding the epidermal growth factor.

**22.** The cell of Claim 19, wherein said gene encoding a natural signal peptide comprises two moieties: a first moiety situated upstream of the restriction endonuclease cleavage site of said restriction endonuclease recognition site and being derived from Escherichia coli; and a second moiety situated downstream of said restriction endonuclease cleavage site and being artificially synthesized.

**23.** The cell of Claim 19, wherein said gene encoding a natural signal peptide is derived from alkaline phosphatase, and said restriction endonuclease recognition site is the Hind III recognition site situated at the boundary between said gene encoding a natural signal peptide and said gene encoding the epidermal growth factor.

**24.** The cell of Claim 19, wherein said gene encoding a natural signal peptide is derived from $\beta$-lactamase, and said restriction endonuclease recognition site is the Nae I recognition site situated at the boundary between said gene encoding a natural signal peptide and said gene encoding the epidermal growth factor.

**Patentansprüche**

**1.** Rekombinanter DNA-Vektor, umfassend ein Gen, codierend ein natürliches Signalpeptid, abgeleitet von Escherichia coli, und ein Gen, codierend einen menschlichen Epidermiswachstumsfaktor oder einen 21-Leu-Epidermiswachstumsfaktor mit der Aminosäuresequenz von Figur 3 ohne das N-terminale Methionin, wobei das zuerst genannte Gen an das zuletzt genannte Gen ohne irgendwelche Nukleotide dazwischen geknüpft ist, wobei das Genprodukt durch eine in den Escherichia-coli-Zellen vorhandene Signalpeptidase gespalten wird, wodurch die Sekretion des reifen Epidermiswachstumsfaktors aus der Zelle ermöglicht wird.

**2.** Rekombinanter DNA-Vektor nach Anspruch 1, dadurch **gekennzeichnet,** daß der rekombinante DNA-Vektor ein Gen, codierend einen Promotor, ein Gen, codierend die Shine-Dalgarno-Sequenz, das Gen, codierend das natürliche Signalpeptid, und das Gen, codierend den Epidermiswachstumsfaktor umfaßt, wobei der Vektor kein anderes Gen, codierend ein sezernierbares Protein, enthält.

**3.** Rekombinanter DNA-Vektor nach Anspruch 1, dadurch **gekennzeichnet,** daß das Gen, das ein natürliches Signalpeptid codiert, mindestens einen Teil einer künstlich geschaffenen Erkennungsstelle für die Restriktionsendonuklease enthält.

**4.** Rekombinanter DNA-Vektor nach Anspruch 3, dadurch **gekennzeichnet,** daß der stromaufwärtige Teil der Restriktionsschnittstelle der Erkennungsstelle für die Restriktionsendonuklease anders als stromabwärts der Grenze zwischen dem Gen, das das natürliche Signalpeptid codiert, und dem Gen, das den Epidermiswachstumsfaktor codiert, angeordnet ist.

**5.** Rekombinanter DNA-Vektor nach Anspruch 4, dadurch **gekennzeichnet,** daß die Restriktionsschnittstelle an der Grenze zwischen dem Gen, das das natürliche Signalpeptid codiert, und dem Gen, das den Epidermiswachstumsfaktor codiert, angeordnet ist.

**6.** Rekombinanter DNA-Vektor nach Anspruch 3, dadurch **gekennzeichnet,** daß das Gen, das ein natürliches Signalpeptid codiert, zwei Teile umfaßt: einen ersten Teil, der stromaufwärts der Restriktionsschnittstelle der Erkennungsstelle für die Restriktionsendonuklease angeordnet ist und sich von Escherichia coli ableitet, und einen zweiten Teil, der stromabwärts der Restriktionsschnittstelle angeordnet ist und künstlich synthetisiert worden ist.

**7.** Rekombinanter DNA-Vektor nach Anspruch 3, dadurch **gekennzeichnet,** daß das Gen, das ein natürliches Signalpeptid codiert, von der alkalischen Phosphatase abgeleitet ist, und daß die Erkennungsstelle für die Restriktionsendonuklease die Hind-III-Erkennungsstelle ist, die an der Grenze zwischen dem Gen, das ein natürliches Signalpeptid codiert, und dem Gen, das den Epidermiswachstumsfaktor codiert, angeordnet ist.

**8.** Rekombinanter DNA-Vektor nach Anspruch 3, dadurch **gekennzeichnet,** daß das Gen, das ein natürliches Signalpeptid codiert, von β-Lactamase abgeleitet ist, und daß die Erkennungsstelle für die Restriktionsendonuklease die Nae-I-Erkennungsstelle ist, die an der Grenze zwischen dem Gen, das ein natürliches Signalpeptid codiert, und dem Gen, das den Epidermiswachstumsfaktor codiert, angeordnet ist.

**9.** Verfahren zur Erzeugung eines menschlichen Epidermiswachstumsfaktors oder eines 21-Leu-Epidermiswachstumsfaktors mit der Aminosäuresequenz von Figur 3 ohne das N-terminale Methionin, dadurch **gekennzeichnet,** daß man
   (a) Escherichia-coli-Zellen, die mit einem rekombinanten DNA-Vektor, umfassend ein Gen, codierend ein natürliches Signalpeptid, das von Escherichia coli abgeleitet ist, und ein Gen, codierend den Epidermiswachstumsfaktor, wobei das zuerst genannte Gen an das zuletzt genannte Gen ohne irgendwelche Nukleotide dazwischen gebunden ist, transformiert worden sind, züchtet;
   (b) eine in den Escherichia-coli-Zellen vorhandene Signalpeptidase das Genprodukt spalten läßt, um die Sekretion des reifen Epidermiswachstumsfaktors aus der Zelle zu gestatten; und
   (c) den Epidermiswachstumsfaktor in reifer Form gewinnt.

**10.** Verfahren nach Anspruch 9, dadurch **gekennzeichnet,** daß der rekombinante DNA-Vektor ein Gen, codierend einen Promotor, ein Gen, codierend die Shine-Dalgarno-Sequenz, das Gen, das das natürliche Signalpeptid codiert, und das Gen, das den Epidermiswachstumsfaktor codiert, umfaßt, wobei der Vektor kein anderes Gen, das ein sezernierbares Protein codiert, enthält.

**11.** Verfahren nach Anspruch 9, dadurch **gekennzeichnet,** daß das Gen, das ein natürliches Signalpeptid codiert, mindestens einen Teil einer künstlich geschaffenen Erkennungsstelle für eine Restriktionsendonuklease enthält.

**12.** Verfahren nach Anspruch 11, dadurch **gekennzeichnet,** daß der stromaufwärtige Teil der Restriktionsschnittstelle der Erkennungsstelle für die Restriktionsendonuklease anders als stromabwärts der Grenze zwischen dem Gen, das das natürliche Signalpeptid, und dem Gen, das den Epidermiswachstumsfaktor codiert, angeordnet ist.

**13.** Verfahren nach Anspruch 12, dadurch **gekennzeichnet,** daß die Restriktionsschnittstelle an der Grenze zwischen dem Gen, das ein natürliches Signalpeptid codiert, und dem Gen, das den Epidermiswachstumsfaktor codiert, angeordnet ist.

**14.** Verfahren nach Anspruch 11, dadurch **gekennzeichnet,** daß das Gen, das ein natürliches Signalpeptid codiert, zwei Teile umfaßt: einen ersten Teil, der stromaufwärts der Restriktionsschnittstelle der Erkennungsstelle für die Restriktionsendonuklease angeordnet ist und sich von Escherichia coli ableitet, und einen zweiten Teil, der stromabwärts der Restriktionsschnittstelle angeordnet ist und künstlich synthetisiert worden ist.

**15.** Verfahren nach Anspruch 11, dadurch **gekennzeichnet,** daß das Gen, das ein natürliches Signalpeptid codiert, sich von der alkalischen Phosphatase ableitet, und daß die Erkennungsstelle für die Restriktionsendonuklease die Hind-III-Erkennungsstelle ist, die an der Grenze zwischen dem Gen, das ein natürliches Signalpeptid codiert, und dem Gen, das den Epidermiswachstumsfaktor codiert, angeordnet ist.

**16.** Verfahren nach Anspruch 11, dadurch **gekennzeichnet,** daß das Gen, das ein natürliches Signalpeptid codiert, sich von β-Lactamase ableitet, und daß die Erkennungsstelle für die Restriktionsendonuklease die Nae-I-Erkennungsstelle ist, die an der Grenze zwischen dem Gen, das ein natürliches Signalpeptid codiert, und dem Gen, das den Epidermiswachstumsfaktor codiert, angeordnet ist.

**17.** Zelle mit der Fähigkeit, einen menschlichen Epidermiswachstumsfaktor oder einen 21-Leu-Epidermiswachstumsfaktor mit der Aminosäuresequenz von Figur 3 ohne das N-terminale Methionin zu erzeugen, dadurch **gekennzeichnet,** daß die Zelle mit einem rekombinanten DNA-Vektor, umfassend ein Gen, codierend ein natürliches Signalpeptid, abgeleitet von Escherichia coli, und ein Gen, codierend den Epidermiswachstumsfaktor, transformiert worden ist, wobei das zuerst genannte Gen an das zuletzt genannte Gen ohne irgendwelche Nukleotide dazwischen gebunden ist, wobei das Genprodukt durch eine in den Escherichia-coli-Zellen vorhandene Signalpeptidase gespalten wird, wodurch die Sekretion des reifen Epidermiswachstumsfaktors aus der Zelle ermöglicht wird.

**18.** Zelle nach Anspruch 17, dadurch **gekennzeichnet,** daß der rekombinante DNA-Vektor ein Gen, codierend einen Promotor, ein Gen, codierend die Shine-Dalgarno-Sequenz, das Gen, codierend das natürliche Signalpeptid, und das Gen, codierend den Epidermiswachstumsfaktor, enthält, wobei der Vektor kein anderes Gen, das ein sezernierbares Protein codiert, enthält.

**19.** Zelle nach Anspruch 17, dadurch **gekennzeichnet,** daß das Gen, das ein natürliches Signalpeptid codiert, mindestens einen Teil einer künstlich geschaffenen Erkennungsstelle für eine Restriktionsendonuklease enthält.

**20.** Zelle nach Anspruch 19, dadurch **gekennzeichnet,** daß der stromaufwärtige Teil der Restriktionsschnittstelle der Erkennungsstelle für die Restriktionsendonuklease anders als stromabwärts der Grenze zwischen dem Gen, codierend ein natürliches Signalpeptid, und dem Gen, codierend den Epidermiswachstumsfaktor, angeordnet ist.

**21.** Zelle nach Anspruch 20, dadurch **gekennzeichnet,** daß die Restriktionsschnittstelle an der Grenze zwischen dem Gen, codierend ein natürliches Signalpeptid, und dem Gen, codierend den Epidermiswachstumsfaktor, angeordnet ist.

**22.** Zelle nach Anspruch 19, dadurch **gekennzeichnet,** daß das Gen, codierend ein natürliches Signalpeptid, zwei Anteile umfaßt: einen ersten Teil, der stromaufwärts der Restriktionsschnittstelle der Erkennungsstelle für die Restriktionsendonuklease angeordnet ist und sich von Escherichia coli ableitet, und einen zweiten Teil, der stromabwärts der Restriktionsschnittstelle angeordnet ist und künstlich synthetisiert worden ist.

**23.** Zelle nach Anspruch 19, dadurch **gekennzeichnet,** daß das Gen, das ein natürliches Signalpeptid codiert, sich von der alkalischen Phosphatase ableitet, und daß die Erkennungsstelle für die Restriktionsendonuklease die Hind-III-Erkennungsstelle ist, die an der Grenze zwischen dem Gen, codierend ein natürliches Signalpeptid, und dem Gen, codierend den Epidermiswachstumsfaktor, angeordnet ist.

**24.** Zelle nach Anspruch 19, dadurch **gekennzeichnet,** daß das Gen, das ein natürliches Signalpeptid codiert, sich von β-Lactamase ableitet, und daß die Erkennungsstelle für die Restriktionsendonuklease die Nae-I-Erkennungsstelle ist, die an der Grenze zwischen dem Gen, codierend ein natürliches Signalpeptid, und dem Gen, codierend den Epidermiswachstumsfaktor, angeordnet ist.

**Revendications**

**1.** Vecteur d'ADN recombinant comprenant un gène codant pour un peptide signal naturel issu d'Escherichia coli et un gène codant pour un facteur de croissance épidermique humain, ou un facteur de croissance épidermique 21-Leu, ayant la séquence d'acides aminés de la Fig. 3 moins la méthionine N-terminale, le premier gène étant lié au second gène sans aucun(s) nucléotide(s) intercalaire(s) entre eux, ledit produit génique étant clivé par une peptidase signal présente dans les cellules d'Escherichia coli, permettant ainsi la sécrétion du facteur de croissance épidermique mature hors de la cellule.

2. Vecteur d'ADN recombinant selon la revendication 1, dans lequel ledit vecteur d'ADN recombinant comprend un gène codant pour un promoteur, un gène codant pour la séquence de Shine-Dalgarno, ledit gène codant pour le peptide signal naturel, et ledit gène codant pour le facteur de croissance épidermique, ledit vecteur ne contenant pas d'autre gène codant pour une protéine excrétable.

3. Vecteur d'ADN recombinant selon la revendication 1, dans lequel ledit gène codant pour un peptide signal naturel comprend au moins une partie d'un site de reconnaissance par une endonucléase de restriction, créé artificiellement.

4. Vecteur d'ADN recombinant selon la revendication 3, dans lequel la partie en amont du site de clivage par une endonucléase de restriction dudit site de reconnaissance par une endonucléase de restriction est située autrement qu'en aval de la limite entre ledit gène codant pour un peptide signal naturel et ledit gène codant pour le facteur de croissance épidermique.

5. Vecteur d'ADN recombinant selon la revendication 4, dans lequel ledit site de clivage par une endonucléase de restriction est situé à la limite entre ledit gène codant pour un peptide signal naturel et ledit gène codant pour le facteur de croissance épidermique.

6. Vecteur d'ADN recombinant selon la revendication 3, dans lequel ledit gène codant pour un peptide signal naturel comprend deux fractions :
   - une première fraction située en amont du site de clivage par une endonucléase de restriction dudit site de reconnaissance par une endonucléase de restriction et étant issu d'Escherichia coli ; et
   - une seconde fraction située en aval dudit site de clivage par une endonucléase de restriction et étant synthétisé artificiellement.

7. Vecteur d'ADN recombinant selon la revendication 3, dans lequel ledit gène codant pour un peptide signal naturel est issu de la phosphatase alcaline, et ledit site de reconnaissance par une endonucléase de restriction est le site de reconnaissance par Hind III situé à la limite entre ledit gène codant pour un peptide signal naturel et ledit gène codant pour le facteur de croissance épidermique.

8. Vecteur d'ADN recombinant selon la revendication 3, dans lequel ledit gène codant pour un peptide signal naturel est issu de la $\beta$-lactamase, et ledit site de reconnaissance par une endonucléase de restriction est le site de reconnaissance par Nae I situé à la limite entre ledit gène codant pour un peptide signal naturel et ledit gène codant pour le facteur de croissance épidermique.

9. Procédé de fabrication d'un facteur de croissance épidermique humain ou d'un facteur de croissance épidermique 21-Leu, ayant la séquence d'acides aminés de la Fig. 3 moins la méthionine N-terminale, ledit procédé comprenant les étapes consistant à :
   (a) cultiver des cellules d'Escherichia coli qui ont été transformées par un vecteur d'ADN recombinant qui comprend un gène codant pour un peptide signal naturel issu d'Escherichia coli et un gène codant pour le facteur de croissance épidermique, le premier gène étant lié au second gène sans aucun(s) nucléotide(s) intercalaire(s) entre eux ;
   (b) amener une peptidase signal présente dans les cellules d'Escherichia coli à cliver ledit produit génique, afin de permettre la sécrétion du facteur de croissance épidermique mature hors de la cellule ; et
   (c) récupérer ledit facteur de croissance épidermique dans une forme mature.

10. Procédé selon la revendication 9, dans lequel ledit vecteur d'ADN recombinant comprend un gène codant pour un promoteur, un gène codant pour la séquence de Shine-Dalgarno, ledit gène codant pour le peptide signal naturel, et ledit gène codant pour le facteur de croissance épidermique, ledit vecteur ne contenant pas d'autre gène codant pour une protéine excrétable.

EP 0 170 266 B1

**11.** Procédé selon la revendication 9, dans lequel ledit gène codant pour un peptide signal naturel comprend au moins une partie d'un site de reconnaissance par une endonucléase de restriction, créé artificiellement.

**12.** Procédé selon la revendication 11, dans lequel la partie en amont du site de clivage par une endonucléase de restriction dudit site de reconnaissance par une endonucléase de restriction est située autrement qu'en aval de la limite entre ledit gène codant pour un peptide signal naturel et ledit gène codant pour le facteur de croissance épidermique.

**13.** Procédé selon la revendication 12, dans lequel ledit site de clivage par une endonucléase de restriction est situé à la limite entre ledit gène codant pour un peptide signal naturel et ledit gène codant pour le facteur de croissance épidermique.

**14.** Procédé selon la revendication 11, dans lequel ledit gène codant pour un peptide signal naturel comprend deux fractions :
- une première fraction située en amont du site de clivage par une endonucléase de restriction dudit site de reconnaissance par une endonucléase de restriction et étant issu d'Escherichia coli ; et
- une seconde fraction située en aval dudit site de clivage par une endonucléase de restriction et étant synthétisée artificiellement.

**15.** Procédé selon la revendication 11, dans lequel ledit gène codant pour un peptide signal naturel est issu de la phosphatase alcaline, et ledit site de reconnaissance par une endonucléase de restriction est le site de reconnaissance par Hind III situé à la limite entre ledit gène codant pour un peptide signal naturel et ledit gène codant pour le facteur de croissance épidermique.

**16.** Procédé selon la revendication 11, dans lequel ledit gène codant pour un peptide signal naturel est issu de la $\beta$-lactamase, et ledit site de reconnaissance par une endonucléase de restriction est le site de reconnaissance par Nae I situé à la limite entre ledit gène codant pour un peptide signal naturel et ledit gène codant pour le facteur de croissance épidermique.

**17.** Cellule capable de produire un facteur de croissance épidermique humain ou un facteur de croissance épidermique 12-Leu, ayant la séquence d'acides aminés de la Fig. 3 moins la méthionine N-terminale, dans laquelle ladite cellule est transformée par un vecteur d'ADN recombinant qui comprend un gène codant pour un peptide signal naturel issu d'Escherichia coli et un gène codant pour le facteur de croissance épidermique, le premier gène étant lié au second gène sans aucun(s) nucléotide(s) intercalaire(s) entre eux, ledit produit génique étant clivé par une peptidase signal présente dans les cellules d'Escherichia coli, permettant ainsi la sécrétion du facteur de croissance épidermique mature hors de la cellule.

**18.** Cellule selon la revendication 17, dans laquelle ledit vecteur d'ADN recombinant comprend un gène codant pour un promoteur, un gène codant pour la séquence de Shine-Dalgarno, ledit gène codant pour le peptide signal naturel, et ledit gène codant pour le facteur de croissance épidermique, ledit vecteur ne contenant pas d'autre gène codant pour une protéine excrétable.

**19.** Cellule selon la revendication 17, dans laquelle ledit gène codant pour un peptide signal naturel comprend au moins une partie d'un site de reconnaissance par une endonucléase de restriction, créé artificiellement.

**20.** Cellule selon la revendication 19, dans laquelle la partie en amont du site de clivage par une endonucléase de restriction dudit site de reconnaissance par une endonucléase de restriction est située autrement qu'en aval de la limite entre ledit gène codant pour un peptide signal naturel et ledit gène codant pour le facteur de croissance épidermique.

**21.** Cellule selon la revendication 20, dans laquelle ledit site de clivage par une endonucléase de restriction est situé à la limite entre ledit gène codant pour un peptide signal naturel et ledit gène codant pour le facteur de croissance épidermique.

28

**22.** Cellule selon la revendication 19, dans laquelle ledit gène codant pour un peptide signal naturel comprend deux fractions :
- une première fraction située en amont du site de clivage par une endonucléase de restriction dudit site de reconnaissance par une endonucléase de restriction et étant issu d'Escherichia coli ; et
- une seconde fraction située en aval dudit site de clivage par une endonucléase de restriction et étant synthétisée artificiellement.

**23.** Cellule selon la revendication 19, dans laquelle ledit gène codant pour un peptide signal naturel est issu de la phosphatase alcaline, et ledit site de reconnaissance par une endonucléase de restriction est le site de reconnaissance par Hind III situé à la limite entre ledit gène codant pour un peptide signal naturel et ledit gène codant pour le facteur de croissance épidermique.

**24.** Cellule selon la revendication 19, dans laquelle ledit gène codant pour un peptide signal naturel est issu de la β-lactamase, et ledit site de reconnaissance par une endonucléase de restriction est le site de reconnaissance par Nae I situé à la limite entre ledit gène codant pour un peptide signal naturel et ledit gène codant pour le facteur de croissance épidermique.

# FIG. 1

# FIG. 5

# FIG. 2

## FIG. 3

```
      Met↓ Asn Ser Asp Ser Glu Cys Pro Leu Ser His Asp Gly
          ——————————17——————————              ——————17——————
      A T G A A T T C C G A C A G C G A G T G T C C G T T G A G T C A C G A C G G C
              EcoRI                                    Hinf I
      T A C T T A A G G C T G T C G C T C A C A G G C A A C T C A G T G C T G C C G
                          ——————————17——————————              ————17————


      Tyr  Cys  Leu His Asp Gly Val  Cys (Leu)  Tyr Ile Glu  Ala Leu Asp Lys
          15                       15      Met         ——17——
      T A C T G T C T G C A C G A T G G C G T T T G C A T G T A T A T T G A A G C T C T G G A C A A A
                                                                        Alu I
      A T G A C A G A C G T G C T A C C G C A A A C G T A C A T A T A A C T T C G A G A C C T G T T T
                    ————————14————————        ——— G ——15———              ————17————


      Tyr  Ala  Cys Asn Cys Val Val  Gly Tyr Ile Gly Glu  Arg Cys Gln Tyr
          15                       17                   14
      T A C G C T T G T A A C T G T G T T G T T G G C T A C A T C G G T G A A C G C T G C C A G T A C
                                                                          Rsa I
      A T G C G A A C A T T G A C A C A A C A A C C G A T G T A G C C A C T T G C G A C G G T C A T G
                      ————————17————————            ————15————              ——15——


      Arg  Asp Leu Lys Trp Trp Glu  Leu Arg End★
          17                       15              ——— 10 ———
      A G A G A T C T G A A A T G G T G G G A A C T G C G T T A G T C G A C C C
            Bgl II                                    Sal I
      T C T C T A G A C T T T A C C A C C C T T G A C G C A A T C A G C T G G G
                  ————————17————————              ————17————

                ——— 17 ———              ——————17——————         Met
      G G G A A G C T T T C A C G T A A A A A G G G T A T C G A C A A T G A A T T C C G A
            Hind III                            Taq I        EcoRI
      C C C T T C G A A A G T G C A T T T T T C C C A T A G C T G T T A C T T A A G G C T
            ——— 10 ———              ————17————              ————15————
```

# FIG. 4

# FIG. 6

# FIG. 7

# FIG. 8

# FIG. 9

FIG. 10

# FIG. 11

# FIG. 12

# FIG. 13

# FIG. 14

# FIG.15

EP 0 170 266 B1

# FIG. 16

44

# FIG.17

FIG.18

EP 0 170 266 B1

FIG. 19